# EUROPÄISCHE PATENTSCHRIFT

(11) **EP 1 287 355 B1**
(45) Veröffentlichungstag und Bekanntmachung des Hinweises auf die Patenterteilung: **21.04.2004**
(21) Anmeldenummer: 01953168.0
(22) Anmeldetag: 11.06.2001
(51) Int. Cl.: G01N 33/50

(54) **VERFAHREN UND VERBINDUNGEN ZUR BEEINFLUSSUNG VON BETA3-INTEGRIN-ABHÄNGIGEN INTRAZELLULÄREN PROZESSEN**
METHODS AND COMPOUNDS FOR INFLUENCING BETA3 INTEGRIN-DEPENDENT INTRACELLULAR PROCESSES
PROCEDES ET COMPOSES DESTINES A INFLUENCER DES PROCESSUS CELLULAIRES DEPENDANT DE LA BETA3-INTEGRINE

(30) Priorität: 09.06.2000 DE 10028645; 28.07.2000 DE 10037272
(43) Veröffentlichungstag der Anmeldung: 05.03.2003
(73) Patentinhaber: Procorde GmbH, 82152 Martinsried (DE)
(72) Erfinder: GAWAZ, Meinrad, 80805 München (DE); UNGERER, Martin, 80799 München (DE); KOLANUS, Waldemar, 81245 München (DE)
(74) Vertreter: Hartz, Nikolai F., Dr.
(86) Internationale Anmeldenummer: PCT/EP2001/006588
(87) Internationale Veröffentlichungsnummer: WO 2001/094613

(56) Entgegenhaltungen:
- SHATTIL, S.J. ET AL: "Beta3-endonexin, a novel polypeptide that interacts specifically with the cytoplasmic tail of the integrin Beta3 subunit" THE JOURNAL OF CELL BIOLOGY, Bd. 131, Nr. 3, November 1995 (1995-11), Seiten 807-816, XP001026503 in der Anmeldung erwähnt
- EIGENTHALER, M. ET AL: "Sites of interaction between the Beta3-integrin cytoplasmic tail and a specific binding partner, Beta3-endonexin" CIRCULATION, Bd. 94, Nr. 8 suppl, 1996, Seite 1355 XP001026820
- KASHIWAGI, H. ET AL: "Affinity modulation of the platelet fibrinogen receptor by Beta3-endonexin, a selective binding partner of the Beta3-integrin cytoplasmic tail" BLOOD, Bd. 88, Nr. 10 Suppl. 1 part 1-2, 6. Dezember 1996 (1996-12-06), Seite 140A XP001026509
- OHTOSHI, A. ET AL: "Beta3-endonexin as a novel inhibitor of cyclin A-associated kinase" BIOCHEMICAL AND BIOPHYSICAL RESEARCH COMMUNICATIONS, Bd. 267, Nr. 3, 27. Januar 2000 (2000-01-27), Seiten 947-952, XP001037980
- EIGENTHALER, M. ET AL: "A conserved sequence motif in the integrin Beta3 cytoplasmic domain is required for its specific interaction with Beta3-endonexin" THE JOURNAL OF BIOLOGICAL CHEMISTRY, Bd. 272, Nr. 12, 21. März 1997 (1997-03-21), Seiten 7693-7698, XP001037974 in der Anmeldung erwähnt
- KASHIWAGI, H. ET AL: "Affinity modulation of platelet integrin Alfa IIb Beta3 by Beta3-endonexin, a selective binding partner of the Beta3 integrin cytoplasmic tail" THE JOURNAL OF CELL BIOLOGY, Bd. 137, Nr. 6, 16. Juni 1997 (1997-06-16), Seiten 1433-1443, XP001037975
- DEDHAR, S.; HANNIGAN, G. E.: "Integrin cytoplasmic interactions and bidirectional transmembrane signalling" CURRENT OPINION IN CELL BIOLOGY, Bd. 8, Nr. 5, Oktober 1996 (1996-10), Seiten 657-669, XP000673950

## Beschreibung

Die vorliegende Erfindung betrifft die Beeinflussung von β3-Integrin-abhängigen intrazellulären Prozessen zum Zweck einer Behandlung von akuten und chronischen Gefäßerkrankungen. Insbesondere betrifft die vorliegende Erfindung neue Verwendungen der bekannten Proteine β3-Endonexin-short und β3-Endonexin-long zum Auffinden von Wirkstoffen sowie spezielle Verfahren zur Identifizierung von Wirkstoffen für eine Behandlung von akuten und chronischen Gefäßerkrankungen. Die Erfindung betrifft ferner Testmischungen zur Identifizierung von Wirkstoffen und spezielle Bestecke (Kit-of-parts) zur Identifizierung von Wirkstoffen. Die Erfindung betrifft auch Arzneimittel, die Wirkstoffe zur Beeinflussung von β3-Integrin-abhängigen intrazellulären Prozessen zum Zweck einer Behandlung von akuten und chronischen Gefäßerkrankungen enthalten sowie Heilverfahren, die solche Arzneimittel einsetzen.

Integrine sind α/β heterodimere Oberflächenproteine und gehören zu einer Klasse von Adhäsionsrezeptoren, die Wechselwirkungen zwischen Zellen oder zwischen Zellen und der extrazellulären Matrix vermitteln.

β3-Integrine bilden eine Unterklasse der Integrine und stellen dynamische Verbindungen zwischen Zellen und der extrazellulären Matrix bereit, wobei ein Informationstransfer beteiligt ist zwischen folgenden zwei Schlüsselregionen des β3-Integrins: den Bindungsdomänen des extrazellulären Liganden und den intrazellulären zytoplasmatischen Rezeptordomänen. Die Signalübertragung über β3-Integrine ist bidirektionell. "Inside-Out"-Signale regulieren die Affinität des β3-Integrins für den Liganden und kontrollieren die Zelladhäsion. "Outside-In"-Signale gehen von der β3-Integrin-Bindung an die extrazelluläre Matrix aus und regulieren viele fundamentale intrazelluläre Prozesse. Dazu zählen das Überleben und die Vermehrung der Zelle, die zelluläre Differenzierung, Morphogenese, Zellwanderung, Phagozytose, Gentranskription und die Integrin-Lokalisierung.

β3-Integrin ist ein Bestandteil des Glykoprotein IIb/IIIa (GP IIb/IIIa, Fibrinogenrezeptor) und des Vitronektinrezeptors. Das Glykoprotein IIb/IIIa hat eine Bedeutung für die Funktion von, inbesondere aktivierten, Thrombozyten und spielt bei akuten Herz-Kreislauf-Erkrankungen, wie beispielsweise bei akuter Koronarthrombose eine wichtige Rolle. Der Vitronektinrezeptor ist an der Adhäsion und der Migration von Endothelzellen beteiligt und spielt bei chronischen Herz-Kreislauf-Erkrankungen, wie beispielsweise der Endothelaktivierung bei Atherosklerose oder in instabilen Plaques eine wichtige Rolle.

Ferner spielen β3-Integrine bei der Hämostase, Thrombose, Wundheilung und Angiogenese eine wichtige Rolle und werden von vielen Gefäßzellen z.B. Blutplättchen, Neutrophile, Endothelialzellen und Zellen der glatten Muskulatur hoch exprimiert. β3-Integrine zeigen sich als Modulatoren einer Reihe von zellulären Funktionen, z.B. als Modulatoren des Zellwachstums, von intrazellulären Signal-Übertragungsmechanismen und der Genregulation.

Aus dem Stand der Technik ist bekannt die Ligandenbindung an Proteine der β3-Integrin enthaltenden Rezeptorfamilie zu antagonisieren. Es existieren bereits mehrere Medikamente, die sogenannten "GPIIb/IIIa-Antagonisten", wie z.B. Abciximab, RGD-Peptide, Fibane und Fibatide, die bei der Behandlung von akuten und chronischen Koronarsyndromen eingesetzt werden. Die Wirksamkeit solcher bekannter GPIIb/IIIa-Antagonisten wird jedoch dadurch begrenzt, dass nach einer Blockade der vorhandenen Rezeptoren weitere Rezeptorproteine aus intrazellulären Speichern auf die Zellmembran externalisieren. Andererseits kann die verabreichte Dosis solcher GPIIb/IIIa-Antagonisten nur in engen Grenzen erhöht werden, weil GPIIb/IIIa-Antagonisten in hohen Konzentrationen zu schwerwiegenden Nebenwirkungen führen können. Die Steuerung der Externalisierung von Rezeptorproteinen der β3-Integrin enthaltenden Rezeptorfamilie ist aus dem Stand der Technik nicht bekannt.

Es ist daher Aufgabe der Erfindung Mittel bereitzustellen, die die Beeinflussung von β3-Integrin-abhängigen intrazellulären Prozessen erlauben, und somit
- eine Stabilisierung bzw. Verbesserung der Wirksamkeit von GPIIb/IIIa-Antagonisten ermöglichen,
- sowie eine Behandlung von Krankheiten zu ermöglichen, deren Entstehung und/oder Verlauf mit β3-Integrin-abhängigen intrazellulären Prozessen in Zusammenhang stehen und die durch eine Regulation (Hemmung oder Förderung) von β3-Integrin-abhängigen intrazellulären Prozessen therapiert werden können.

Es ist weiterhin Aufgabe der Erfindung, neue Verwendungen von speziellen Proteinen beim Auffinden von Wirkstoffen zur Behandlung von Gefäßerkrankungen bereitzustellen.

Ferner ist es Aufgabe der vorliegenden Erfindung, Verfahren bereitzustellen mit denen Wirkstoffe aufgefunden werden können, die geeignet sind in Kombination mit bekannten GPIIb/IIIa-Antagonisten eingesetzt zu werden und deren Wirksamkeit sichern oder erhöhen.

Ferner ist es Aufgabe der Erfindung Arzneimittel bereitzustellen, die identifizierte Wirkstoffe enthalten sowie Verfahren zur Behandlung von akuten bzw. chronischen Gefäßerkrankungen.

Diese Aufgaben werden gemäß der Ansprüche gelöst. Die Erfindung beruht auf dem besonderen technischen Merkmal der Beeinflussung von β3-Integrin-abhängigen intrazellulären Prozessen zum Zweck einer Behandlung von akuten und chronischen Gefäßerkrankungen durch den antagonistischen Wirkungszusammenhang der bekannten Proteine β3-Endonexin-short und β3-Endonexin-long (Genbank accession no. HS371391). Die Proteine sind beispielsweise aus Shattil S. J. *et al*., J. Cell Biology, 131, (1995), 807-816 sowie Genbank, Nr. U37139 vom 5. 12. 1995 bekannt. Speziell beruht die Erfindung auf der Tatsache, dass überraschenderweise die bekannten Proteine β3-Endonexin-short und β3-Endonexin-long spezifisch die Internalisierung sowie Signalweiterleitung von GPIIb/IIIa und Vitronektinrezeptoren steuern. Die Steuerung der Internalisierung erfolgt über einen Antagonismuns der Wirkungen von β3-Endonexin-short und β3-Endonexin-long, wobei β3-Endonexin-short eine Internalisierung und das Recycling beider Rezeptoren fördert und β3-Endonexin-long eine Internalisierung der Rezeptoren inhibiert. Insbesondere wurde gefunden, dass β3-Endonexin-short spezifisch die Internalisierung und das Recycling von GPIIb/IIIa an menschlichen Thrombozyten und von Vitronektinrezeptoren an menschlichen Endothelzellen fördert. Außerdem können über β3-Endonexin-short verschiedene nukleäre DNA-Transkriptionspromotoren für physiologisch wichtige Mediatoren hoch- oder herunterreguliert werden. Andererseits wurde gefunden, dass β3-Endonexin-long als Antagonist die Internalisierung beider Rezeptoren blockiert.

Außerdem wurde gefunden, dass β3-Endonexin-short und -long große Bedeutung für die intrazelluläre Signaltransduktion von Vitronektinrezeptoren in menschlichen Endothelzellen, für die Aktivierung von Endothelzellen; für die nukleäre Transkription proatherogener Mediatoren und damit generell für den Prozess der Atherogenese und Plaque-Destabilisierung haben.

β3-Endonexin-short und β3-Endonexin-long regulieren auch die Oberflächenexpression oder die Sekretion von Signalstoffen, die für Herzkreislauferkrankungen (akute Thrombose, Atherosklerose, Plaque-Ruptur) von entscheidender Bedeutung sind.

Ein in beiden Integrin-Rezeptoren konserviertes Peptid-Sequenzmotiv ist entscheidend für die Bindung von β3-Endonexin-short an beide Rezeptoren. Die Bindungsspezifität zwischen β3-Endonexin-short und der zytoplasmatischen Domäne von β3-Integrin beruht auf einem Membran-distalen NITY-Motiv innerhalb der zytoplasmatischen Domäne von β3-Integrin. β3-Endonexin-long (20 kDa) dagegen inhibiert die β3-Endonexin-short-vermittelte Integrin-Rezeptor-Internalisierung.

Die vorliegende Erfindung bietet den Vorteil, dass über eine Intervention an β3-Endonexin die Wirkung von bereits existierenden Medikamenten gesichert bzw. verbessert werden kann. Insbesondere ist es nunmehr möglich, sowohl durch existierende Medikamente (z.B. Abciximab, RGD-Peptide, Fibane, Fibatide) die externe Ligandenbindung an GPIIb/IIIa und an Vitronektinrezeptoren zu beeinflussen, als auch erfindungsgemäß deren Intemalisierungs-abhängige intrazelluläre Effekte zu beeinflussen. Vorteilhafterweise kann mit einer Steuerung von β3-Endonexin-short und -long spezifisch die Oberflächenexpression, die Internalisierung und die Signaltransduktion beider Rezeptoren gesteuert werden, wobei β3-Endonexin-short die Internalisierung fördert, während β3-Endonexin-long die Internalisierung blockiert.

Ausgehend von dieser Erkenntsnis ist es damit nicht nur möglich die Wirksamkeit bereits existierender Medikamente zu sichern bzw. zu verbessern, sondern es ist nunmehr auch möglich einerseits spezifisch die Zusammensetzung der thrombozytären alpha-Granula, d.h. deren Mediatoren-Wiederaufnahme-Kapazität und deren sekretorische Eigenschaften zu beeinflussen. Andererseits kann mit Endonexin die Aktivierung von Endothelzellen ebenfalls über die Vitronektinrezeptor-Internalisierung beeinflusst werden.

Damit sind β3-Endonexin-short und β3-Endonexin-long entscheidende Signaltransduktionsmoleküle sowie Zielproteine zur Entwicklung neuer pharmakologischer Wirkstoffe bei akuten und chronischen Gefäßprozessen.

Die vorliegende Erfindung stellt daher eine Verwendung von β3-Endonexin-long oder β3-Endonexin-short bereit zum Auffinden von Wirkstoffen zur Behandlung von Arteriosklerose, daraus resultierenden instabilen Plaques, akuter Koronarthrombose, Herzinfarkt, Schlaganfall, peripheren arteriellen Verschlußkrankheiten, chronischem venösen Ulcus und Restenosierungsprozessen.

Ausserdem stellt die vorliegende Erfindung ein Verfahren zur Identifizierung von Verbindungen bereit, die eine Bindung von β3-Endonexin-short an β3-Integrin hemmen, das durch die folgenden Schritte gekennzeichnet ist:
(a) Inkubation eines Gemisches enthaltend
   (a1) β3-Endonexin-short oder ein hierzu in Bezug auf die Bindung an β3-Integrin funktionsäquivalentes Peptid oder Polypeptid;
   (a2) β3-Integrin oder ein hierzu in Bezug auf die Bindung an β3-Endonexinshort funktionsäquivalentes Peptid oder Polypeptid;
   (a3) eine zu testende Verbindung; und
(b) Nachweis der Hemmung einer Bindung der Komponente (a1) an die Komponente (a2) bei Anwesenheit der Verbindung (a3) im Vergleich zur Abwesenheit der Verbindung (a3).

Ausserdem stellt die vorliegende Erfindung ein Verfahren bereit zur Identifizierung von Verbindungen, die eine Bindung von β3-Endonexin-long an β3-Integrin hemmen, gekennzeichnet durch die folgenden Schritte:
(a) Inkubation eines Gemisches, enthaltend
   (a1) β3-Endonexin-long oder eine hierzu in Bezug auf den Einfluss auf β3-Integrin funktionsäquivalentes Peptid oder Polypeptid;
   (a2) β3-Integrin oder ein hierzu in Bezug auf die Wechselwirkung mit β3-Endonexin-long funktionsäquivalentes Peptid oder Polypeptid;
   (a3) eine zu testende Verbindung;
(b) Nachweis der Hemmung eines Einflusses der Komponente (a1) auf die Komponente (a2) bei Anwesenheit der Verbindung (a3) im Vergleich zur Abwesenheit der Verbindung (a3).

Der Einfluss der Komponente (a1) auf die Komponente (a2) kann insbesondere durch eine direkte oder indirekte Bindung erfolgen.

Ausserdem stellt die vorliegende Erfindung ein Verfahren bereit zur Identifizierung von Verbindungen, die die Konkurrenz der Bindung von β3-Integrin an β3-Endonexin-short und β3-Endonexin-long verschieben, gekennzeichnet durch die folgenden Schritte:
(a) Inkubation eines Gemisches, enthaltend
   (a1) β3-Endonexin-short oder ein hierzu in Bezug auf die Bindung an β3-Integrin funktionsäquivalentes Peptid oder Polypeptid;
   (a2) β3-Endonexin-long oder ein hierzu in Bezug auf die Bindung an β3-Integrin funktionsäquivalentes Peptid oder Polypeptid;
   (a3) β3-Integrin oder ein hierzu in Bezug auf die Bindung an β3-Endonexin-short und/oder β3-Endonexin-long funktionsäquivalentes Peptid oder Polypeptid;
   (a4) eine zu testende Verbindung; und
(b) Nachweis einer Verschiebung des Verhältnisses der Bindung der Komponente (a1) an die Komponente (a3) zur Bindung der Komponente (a2) an die Komponente (a3) im Vergleich zur Abwesenheit der zu testenden Verbindung (a4).

Die erfindungsgemäßen Screening-Verfahren können vorteilhafterweise als High-Throughput-Screening (HTS)-Verfahren durchgeführt werden.

HTS-geeigente Screeningtests auf neue "kleine Moleküle", die β3-Endonexin-short inhibieren können beispielhaft dadurch erfolgen, dass kleine Moleküle aus großen Substanzbibliotheken auf ihre Eignung getestet werden, die Bindung von β3-Endonexin-short an GPIIb/IIIa oder den Vitronektinrezeptor zu inhibieren.

Als Assay ist einerseits ein Assay mit einzelnen Proteinkomponenten vorstellbar. Andererseits wäre ein Assay mit, insbesondere stabil, GPIIb/IIIa- und β3-Endonexin-short-exprimierenden CHO-Zellen vorstellbar. Die Art des Assays steht im Belieben des Fachmanns, wobei folgende Möglichkeiten beispielhaft offenbart werden.

Folgende Assays mit einzelnen Proteinkomponenten können speziell genannt werden. In einer Ausführungsform wird β3-Endonexin-short nach Biotinylisierung auf Mikrotiterplatten Avidin-immobilisiert. In einer anderen Ausführungsform wird β3-Endonexin-short mit His-Tag auf Nickelplatten aufgebracht. Die spezifische Bindungsstelle von β3-Endonexin-short an beide Rezeptoren ist bekannt (Eigenthaler, M. *et al*., J. Biol. Chem., 272, (1997), 7693-7698) und wird vorzugsweise als 10er-Peptid, z. B. über eine Cystein-Brücke, an Fluoreszein gekoppelt. Dieses Konstrukt wird in Gegenwart verschiedener Testmoleküle auf der Platte inkubiert, dann gewaschen, um anschließend die Menge verbleibenden Konstrukts anhand der Restfluoreszenz zu quantifizieren. In einer weiteren Ausführungsform kann ein Assay auch mit Hilfe eines Fluoreszenz-Resonanz-Energie-Transfers (FRET) durchgeführt werden. Zu diesem Zweck wird ein Fusionsprotein, das β3-Endonexin-short-Green Fluorescent Protein (GFP) umfasst, wie oben auf Platten immobilisiert. Mit diesem wird das oben erwähnte Konstrukt (10er-Peptid+Fluoreszein) auf der Platte inkubiert. Die Bindung von β3-Endonexin-short an das Indikatorpeptid kann dann anhand eines Wellenlängenshifts des anregenden Blau-Lichtes auf die Emission von Rotlicht. gemessen werden, weil bei einer echten Bindung hochspezifisch ein Resonanzenergietransfer zwischen Fluoreszein und GFP stattfindet.

Ferner kann eine Affinitätssäule mit Substanzen, die potentielle Bindungspartner darstellen könnten für die Zwecke des erfindungsgemäßen Verfahrens eingesetzt werden. Entsprechend werden über Affinitätssäulen mit Substanzen, die potentielle Bindungspartner darstellen können, gereinigte Fusionsproteine von β3-Endonexin-short und GFP gegeben. Nach Waschung wird die verbleibende Restfluoreszenz quantifiziert.

Ein Zell-Assay zur Auffindung von Inhibitoren von β3-Endonexin-short kann speziell folgende Schritte umfassen:
(a) Kultivierung von gemäß den Angaben in Ylänne et al., J Biol Chem 1995; 270: 9550-9557 hergestellten stabil β3-Integrin exprimierenden Chinese-Hamster-Ovary-Zellen (CHO) in herkömmlichen Zellkulturmedien;
(b) Induktion einer Expression von β3-Endonexin-short, vorzugsweise durch liposomalen Gentransfer in diese Zellen;
(c) Zugabe einer zu testenden Verbindung zu dem Kulturmedium der Zellen;
(d) Messung der Oberflächendichte der β3-Integrine an den Zellen mittels Fluoreszenz-aktiviertem Cell Sorting (FACS) mit herkömmlichen spezifischen Antikörpern nach Ablauf einer bestimmten Zeit, beispielsweise 24 Stunden später;
(e) Nachweis der Zunahme der Oberflächendichte in Gegenwart der Substanz aus Stufe (c) im Vergleich zu deren Abwesenheit.

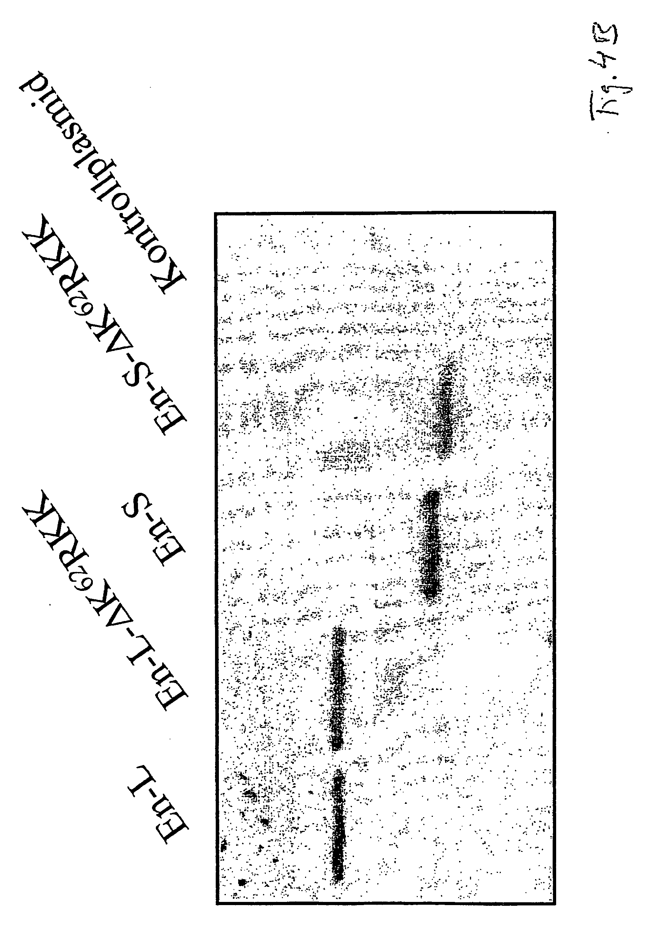

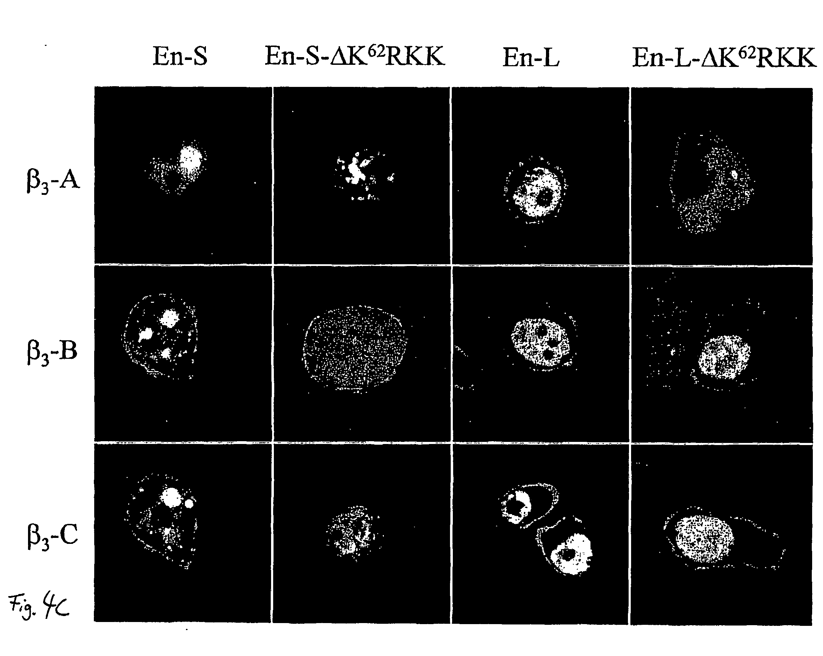

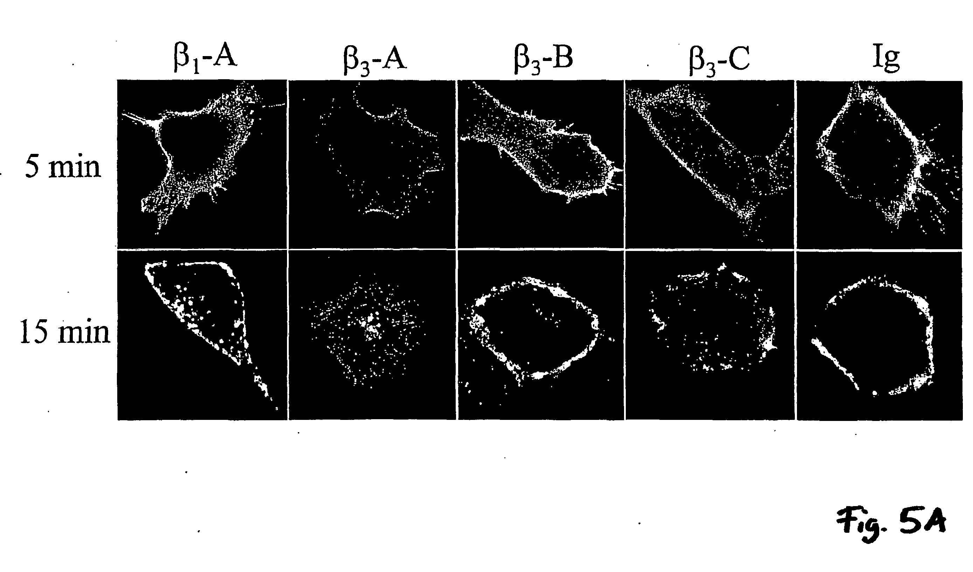

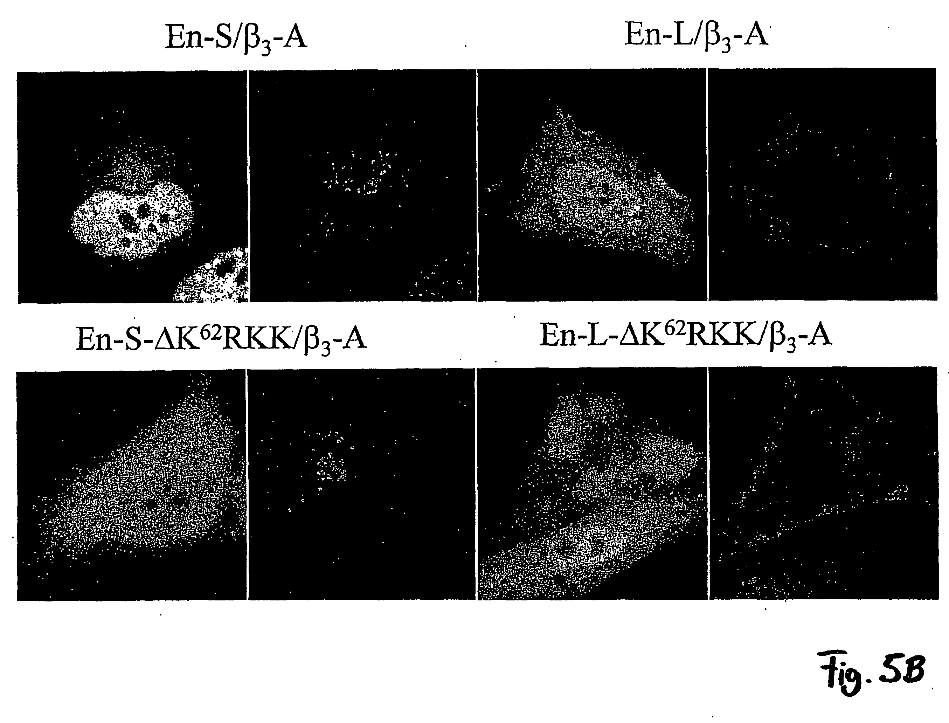

Alternativ kann, wie in Figur 5B beschrieben, die konstitutive Internalisierung von Texas-Red-konjugiertem anti-IgG1-MAK mittels Fluoreszenztechnik bestimmt werden. Hier kann die GPIIb/IIIa-Intemalisierung (Messung über Antikörperbindung und FACS) auf die Inhibition durch kleine Moleküle untersucht werden.

HTS-geeignete Screeningtests auf neue kleine Moleküle, die β3-Endonexin-long inhibieren, können beispielhaft dadurch erfolgen, dass kleine Moleküle aus großen Substanzbanken auf ihre Eignung getestet werden, die Bindung von long an GPIIb/IIIa oder den Vitronektinrezeptor zu inhibieren.

Als Assay ist einerseits ein Assay mit einzelnen Proteinkomponenten vorstellbar. Andererseits wäre ein Assay mit, insbesondere stabil, GPIIb/IIIa- und β3-Endonexin-long-exprimierenden CHO-Zellen vorstellbar. Die Art des Assays steht im Belieben des Fachmanns, wobei folgende Möglichkeiten beispielhaft offenbart werden.

Folgende Assays mit einzelnen Proteinkomponenten können speziell genannt werden. In einer Ausführungsform werden Aptamere oder spezifische Antikörper gegen die nur in β3-Endonexin-long, nicht aber in β3-Endonexin-short vorkommenden Proteinabschnitte, selektiert werden. Dazu werden β3-Endonexin-short und die nur in β3-Endonexin-long vorkommenden Proteindomänen bakteriell überexprimiert und über ein GST-Tag gereinigt. Dann können über die NEB-Technologie spezifische Aptamere oder Antikörper selektiert werden, die nur an dieses Konstrukt, nicht aber an gereinigtes β3-Endonexin-short binden. Aptamere können dann über eine Konversionstechnologie in kleine Moleküle umgewandelt werden, die dann mit den im folgenden beschriebenen Techniken zur Identifizierung von Inhibitoren verwendet werden.

Speziell kann β3-Endonexin-long nach Biotinylisierung auf Mikrotiterplatten Avidin-immobilisiert werden. In einer anderen Ausführungsform kann β3-Endonexin-long mit His-Tag auf Nickelplatten aufgebracht werden. β3-Integrin wird z. B. über eine Cystein-Brücke an Fluoreszein gekoppelt. Dieses Konstrukt wird in Gegenwart verschiedener Testmoleküle auf der Platte inkubiert, dann gewaschen, um anschließend die Menge verbleibenden Konstruktes anhand der Restfluoreszenz zu quantifizieren.

Weiterhin kann ein Assay aber auch mit Hilfe eines Fluoreszenz-Resonanz-Energie-Transfers (FRET) durchgeführt werden. Dazu wird ein Fusionsprotein, das β3-Endonexin-long-Green Fluorescent Protein (GFP) umfasst, wie oben auf Platten immobilisiert. Mit diesem wird das oben erwähnte Konstrukt (β3-Integrin+Fluoreszein) auf der Platte inkubiert. Die Bindung von β3-Endonexin-long an das Integrin kann dann anhand eines Wellenlängenshifts des anregenden Blau-Lichtes auf die Emission von Rotlicht gemessen werden, weil bei einer echten Bindung hochspezifisch ein Resonanzenergietransfer zwischen Fluoreszein und GFP stattfindet.

In einer weiteren Ausführungsform kann eine Affinitätssäule mit Substanzen, die potentielle Bindungspartner darstellen könnten, eingesetzt werden. Speziell werden gereinigte Fusionsproteine aus β3-Endonexin-long und GFP über Affinitätssäulen mit Substanzen gegeben, die potentielle Bindungspartner darstellen könnten. Nach Waschung wird die verbleibende Restfluoreszenz quantifiziert.

Ein Zell-Assay zur Auffindung von Inhibitoren von β3-Endonexin-long kann speziell folgende Schritte umfassen:
(a) Kultivierung von gemäß den Angaben in Ylänne et al., J Biol Chem 1995; 270: 9550-9557 hergestellten stabil β3-Integrin exprimierenden Chinese-Hamster-Ovary-Zellen (CHO) in herkömmlichen Zellkulturmedien;
(b) Induktion einer Expression von β3-Endonexin-long, vorzugsweise durch liposomalen Gentransfer in diese Zellen;
(c) Zugabe einer zu testenden Verbindung zu dem Kulturmedium der Zellen;
(d) Messung der Oberflächendichte der β3-Integrine an den Zellen mittels Fluoreszenz-aktiviertem Cell Sorting (FACS) mit herkömmlichen spezifischen Antikörpern nach Ablauf einer bestimmten Zeit, beispielsweise 24 Stunden später;
(e) Nachweis der Abnahme der Oberflächendichte in Gegenwart der Substanz aus Stufe (c) im Vergleich zu deren Abwesenheit.

Alternativ kann, wie in Figur 5B beschrieben, die konstitutive Intemalisierung von Texas-Red-konjugiertem anti-IgG1-MAK mittels Fluoreszenztechnik bestimmt werden. Hier kann die GPIIb/IIIa-Internalisierung (Messung über Antikörperbindung und FACS) auf die Inhibition durch kleine Moleküle untersucht werden.

Es wurde überraschenderweise gefunden, dass β3-Endonexin die Oberflächenexpression von β3-Integrin und von β3-Integrin-abhängigen intrazellulären Prozessen reguliert und dass sich über β3-Endonexin dieses beeinflussen läßt. Insbesondere läßt sich die Internalisiserung von β3-Integrin, das Oberflächen-Recycling von β3-Integrin und die Zusammensetzung von thrombozytären bzw. endothelialen Speichervesikeln beeinflussen. Desweiteren kann auf die Aktivierung von Promotoren für Oberflächenrezeptor-Gene in Endothelzellen über β3-Endonexin Einfluß genommen werden.

Bei den zu der vorliegenden Erfindung führenden Experimenten wurden Einzelketten-Chimäre mit Varianten und mutierten Formen aller zytoplasmatischen β3-Domänen erzeugt. Nach transienter Transfektion in CHO-Zellen oder Primärkulturen von HUVEC-Zellen zeigte sich, dass die β3-A-Chimären im Vergleich zu den entsprechenden β3-B-oder β3-C-Fusionsproteinen oder schwanzlosen Konstrukten eine stark verringerte Zelloberflächenexpression zeigten, während die "steady state"-Spiegel aller Chimären praktisch identisch waren. Die Untersuchungen mit Mutanten der zytoplasmatischen Domäne zeigten auch, dass das NITY-Motiv bei β3-A (Pos. 756-759) für die Plasmaexpression von β3-A von kritischer Bedeutung ist. Außerdem wurde der Transport von β3-A an die Zelloberfläche durch das zytoplasmatische Protein β3-Endonexin spezifisch moduliert. β3-Endonexine kommen als zwei unterschiedliche Spleißvarianten vor, die durch eine unterschiedliche Fähigkeit bezüglich der Interaktion mit dem β3-Schwanz gekennzeichnet sind, Die Coexpression der nativen langen Form von β3-Endonexin (β3-Endonexin-long; En-L), die nicht mit dem β3-Schwanz interagiert, wirkte als ein dominant negativer Inhibitor der β3-A-Internalisierung. Außerdem war bei der Tyr⁷⁵⁹-Ala-Substrtutionsmutante α_{11b}β3(Y759A) die anti-β3-MAK-induzierte Endozytose des nativen β3-Integrins α_{11b}β3 drastisch verringert und die Expression der langen Isoform von β3-Endonexin (En-L; β3-Endonexin-long) führte zu einer substantiellen Abnahme der Internalisierung von Wildtyp-a_{11b}β3. Somit koppelt β3-Endonexin die β3-A-Isoformen zu einem spezifischen Rezeptor-Recycling-Weg.

Ferner wurde erkannt, dass dieser Recycling-Weg Auswirkungen auf die Mediatoren-Wiederaufnahme-Kapazität von Speichervesikeln im menschlichen Thrombozyten (α, Granula) und Endothelzellen (Weibel-Palade bodies) und somit auf deren Sekretionsverhalten hat. Es ist bekannt, dass die Sekretion von vasoaktiven Substanzen z.B. platelet-derived growth factor (PDGF), endothelial growth factor (EGF), vascular endothelial growth factor (VEGF), verschiedener Chemokine und Cytokine aus thrombozytären α-Granula eine entscheidende Rolle bei akuten und chronischen Gefäßerkrankungen, spielt. Darüber hinaus wurde gefunden, dass über β3-Endonexin in diese Prozesse eingegriffen werden kann. Ferner wurde erkannt, dass mit β3-Endonexin in pro-atherogene Signalwege in menschlichen Endothelzellen, wie die Expression von Monocyte Chemoattractant Protein-1 (MCP-1) und dessen Sekretion aus menschlichen Endothelzellen, oder in die Expression von Urokinase-type Plasminogen Activator Receptor (uPAR) an der Zelloberfläche, der wesentlich deren Migrationsverhalten beeinflusst, eingegriffen werden kann.

Die Förderung von β3-Integrin-Internalisierungs-abhängigen Prozessen bzw. die Hemmung der Sekretion von pro-atherogenen Mediatoren aus Endothelzellen und die Hemmung der Expression von migrationsfördernden Oberflächenrezeptoren auf Endo-thelzellen können einerseits durch eine Hemmung der Expression von β3-Endonexinlong (über Antisense-RNAs oder Ribozyme) oder durch eine Hemmung der Aktivität von β3-Endonexin-long (beispielsweise mittels Antikörper) oder andererseits z. B. durch eine Steigerung der Expression oder der Aktivität von β3-Endonexin-short (durch Applikation des Proteins selbst oder eines das Protein exprimierenden Vektors) erreicht werden. Die Hemmung von β3-Integrin-Internalisierungs-abhängigen Prozessen bzw. die Hemmung der Sekretion von pro-atherogenen Mediatoren aus Thrombozyten kann durch eine Hemmung der Aktivität von β3-Endonexin-short mittels einer die Bindung von β3-Endonexin-short an das NITY-Motiv von β3-Integrin hemmenden Verbindung, z.B. eines gegen das NITY-Motiv gerichteten Antikörpers, erfolgen. Ferner kann die Bindung von β3-Endonexin-short an die zytoplasmatische Domäne des β3-Integrins durch Verabreichung eines das NITY-Motiv enthaltenden Peptids (kompetitiver Antagonist) gehemmt werden.

Der hier verwendete Ausdruck "β3-Endonexin-short" oder "ein eine β3-Endonexin-short kodierende DNA enthaltender Expressionsvektor" betrifft jede Form des Proteins selbst bzw. die von der DNA kodierte Form, die an die zytoplasmatische Domäne von β3Integrin β3-A binden und dadurch β3-Integrin-Internalisierungs-abhängige Prozesse bzw. die Hemmung der Sekretion von pro-atherogenen Mediatoren aus Endotheizellen und die Hemmung der Expression von migrationsfördernden Oberflächenrezeptoren auf Endothelzellen fördern kann, und dazu zählen nicht nur die Wildtypform, sondern auch Formen, die gegenüber der Wildtypform Veränderungen in der Aminosäuresequenz, wie Substitutionen von einer oder mehreren Aminosäuren, Deletionen oder Additionen aufweisen, ohne dass dadurch die ursprüngliche biologische Aktivität wesentlich verändert wind.

Durch die Erniedrigung oder Hemmung der Expression von β3-Endonexin-long, können ebenfalls β3-Integrin-Internalisierungs-abhängige Prozesse gefördert bzw. die Sekretion van pro-atherogenen Mediatoren aus Endothelzellen und die Expression von migrationsfördenden Oberflächenrezeptoren auf Endothelzellen gehemmt werden. Dies kann z. B. durch Ribozyme oder Antisense-RNAs erreicht werden, die die Translation von β3Endonexin-long verringern oder eliminieren. Vorzugsweise sind diese Antisense-RNAs und Ribozyme zu einer kodierenden Region der mRNA komplementär.

Der Fachmann ist in der Lage, ausgehend von den veröffentlichten DNA-Sequenzen von β3-Endonexin-short bzw. β3-Endonexin-long (die betreffenden Sequenzen können der Genbank/EMBL accession number hs371391 entnommen werden) geeignete Antisense-RNAs herzustellen und anzuwenden. Geeignete Vorgehensweisen sind beispielsweise in EB-B1 0 223 399 oder EP-A1 0 458 beschrieben. Ribozyme sind RNA-Enzyme und bestehen aus einem einzelnen RNA-Strang. Diese können andere RNAs intermolekular spalten, beispielsweise die En-L-mRNAs. Diese Ribozyme müssen prinzipiell über zwei Domänen verfügen, (1) eine katalytische Domäne und (2) eine Domäne, die zu der Ziel-RNA komplementär ist und an diese binden kann, was die Voraussetzung für eine Spaltung der Ziel-RNA ist. Ausgehend von in der Literatur beschriebenen Vorgehensweisen ist es inzwischen möglich, spezifisch Ribozyme zu konstruieren, die eine gewünschte RNA an einer bestimmten, vorgewählten Stelle schneiden (siehe beispielsweise Tanne et al., in: Antisense Research und Applications, CRC Press, Inc. (1993), 415-426). Vorzugsweise hybridisieren die vorstehenden Ribozyme oder Antisense-RNAs über einen Bereich von mindestens 15, mehr bevorzugt mindestens 25 und am meisten bevorzugt mindestens 50 Basen mit der En-L-mRNA.

Für die Insertion und Expression der vorstehend diskutierten β3-Endonexin-short kodierenden DNA, Ribozyme oder Anitsense-RNAs können eine Reihe von dem Fachmann bekannten Expressionsvektoren verwendet werden. Die Bezeichnung "Expressionsvektor" bezieht sich auf ein Plasmid (pUC18, pBR322, pBlueScript etc-), auf ein Virus oder ein anderes geeignetes Vehikel. Die vorstehenden DNA-Sequenzen sind im Expressionsvektor mit regulatorischen Elementen funktionell verknüpft, die deren Expression in prokaryotischen oder eukaryotischen Wirtszellen erlauben. Solche Vektoren enthalten neben den regulatorischen Elementen, beispielsweise einem Promotor, typischerweise einen Replikationsursprung und spezifische Gene, die die phänotypisch Selektion einer transformierten Wirtszelle erlauben. Zu den regulatorischen Elementen für die Expression in Prokaryoten, beispielsweise E.coli, zählen der lac-,trp-Promotor oder T7-Promotor, und für die Expression in Eukaryoten der AOX1- oder GAL1-Promotor in Hefe und der CMV-,SV40-, RVSAO-Promotor, CMV-oder SV40- Enhancer für die Expression in tierischen Zellen. Weitere Beispiele für geeignete Promotoren sind der Metallothionein 1- und der Polyhedrin-Promotor. Zu geeigneten Expressionsvektoren für E.coli zählen beispielsweise pGEMEX, pUC-Derivate, pGEX-2T, pET3b und pQE-8, wobei letzterer bevorzugt ist. Zu den für die Expression in Hefe geeigneten Vektoren zählen pY100 und Ycpad1, für die Expression in Säugerzellen pMSXND, pKCR, pEFBOS, cDM8, pCEV4 und die in den nachstehenden Beispielen beschriebenen Vektoren, Zu den geeigneten Expressionsvektoren zählen auch von Baculovirus abgeleitete Vektoren für die Expression in Insektenzellen, beispielsweise pAcSGHisNT-A.

Vorzugsweise werden die vorstehend beschriebenen DNA-Sequenzen in einen für die Gentherapie geeigneten Vektor inseriert, beispielsweise unter Kontrolle eines gewebespezifischen Promotors, und in die Zellen eingeschleust. In einer bevorzugten Ausführungsform ist der die vorstehend beschriebenen DNA-Sequenzen enthaltende Vektor ein Virus, beispielsweise ein Retrovirus, Adenovirus, adenoassoziiertes Virus oder Vaccinia-Virus. Beispiele für geeignete Retroviren sind MoMuLV, HaMuSV, MuMTV, RSV oder GaLV. für Zwecke der Gentherapie können die erfindungsgemallen DNA-Sequenzen auch in Form von kolloidalen Dispersionen zu den Zielzellen transportiert werden. Dazu zählen beispielsweise Liposomen oder Lipoplexe (Mannino et al., Bio-techniques 6 (1988), 682). Außerdem können diese Vektoren nach überlichen Verfahren so modifiziert werden, dass sie zielspezifisch sind. Weitere geeignete Vektoren und Verfahren für die in vitro- oder in vivo-Gentherapie sind in der Literatur beschrieben und dem Fachmann bekannt; siehe z.B. auch WO 93/04701, WO 92/22635, WO 92/20316, WO 92/19749, WO 92/06180, WO 94/29489 und WO 97/00957, Diese Gentherapie kann sowohl für die Forderung von β3-Integrin-Internalisierungs-abhängigen Prozessen bzw. die Hemmung der Sekretion von pro-atherogenen Mediatoren aus Endothelzellen und die Hemmung der Expression von migrationsfördernden Oberflächenrezeptoren auf Endothelzellen (beispielsweise mit einem Vektor, der das Gen für β3-Endonexin-short oder eine DNA-Sequenz für ein anti-β3-Endonexin-long-mRNA-Ribozym enthalt), als auch für eine Hemmung von β3-Integrin-Internalisierungs-abhängigen Prozessen bzw. die Hemmung der Sekretion von pro-atherogenen Mediatoren aus Thrombozyten (beispielsweise mit einem Vektor, der ein Gen für β3-Endonexin-long oder eine DNA-Sequenz enthält, die ein das NITY-Motiv enthaltendes Peptid oder Polypeptid kodiert) Anwendung finden.

Allgemeine, auf dem Fachgebiet bekannte Verfahren können zur Konstruktion von Expressionsvektoren, die die vorstehenden DNA-Sequenzen und geeignete Kontrolisequenzen enthalten, verwendet werden. Zu diesen Verfahren zählen beispielsweise in vitro-Rekombinationstechniken, synthetische Verfahren, sowie in vivo-Rekombinationsverfahren, wie sie beispielsweise in Sambrook et al., supra, beschrieben sind.

Das in dem erfindungsgemäßen Arzneimittel enthaltene β3-Endonexin-short wird vorzugsweise rekombinant hergestellt, z.B. mittels den vorstehend beschriebenen Expressionsvektoren. Zu geeigneten Wirtszellen zählen z.B. Bakterien (beispielsweise die E.coli-Stämme HB101, DH1, x1776, JM101, JM109, BL21 und SG13009), Hefe, vorzugsweise S. cerevisiae, Insektenzellen, vorzugsweise sf9-Zellen, und Tierzellen, vorzugsweise Säugerzellen. Bevorzugte Säugerzellen sind CHO-, VERO, BHK-, HeLa-COS-, MDCK, 293- und W138-Zellen. Verfahren zur Transformation dieser Wirtszellen, zur phänotypischen Selektion von Transformanten und zur Expression der DNA-Sequenzen unter Verwendung der vorstehend beschriebenen Expressionsvektoren sind auf dem Fachgebiet bekannt. Dem Fachmann sind auch Bedingungen bekannt, transformierte Wirtszellen zu kultivieren. Geeignete Reinigungsverfahren (beispielsweise präparative Chromatographie, Affinitätschromatographie, beispielsweise Immunoaffinitätschromatographie, HPLC etc.) sind ebenfalls allgemein bekannt.

Der hier verwendete Begriff "anti-β3-Endonexin-long-Antikörper" betrifft Antikörper oder Fragmente davon, die an β3-Endonexin-long spezifisch binden, nicht jedoch an β3-Endonexin-short. Diese Antikörper (und die weiteren in Zusammenhang mit dem erfindungsgemäßen Arzneimittel beschriebenen Antikörper) können monoklonale, polyklonale oder synthetische Antikörper sein oder Fragmente davon. In diesem Zusammenhang bedeutet der Begriff "Fragment" alle Teile des monoklonalen Antikörpers (z.B. Fab-, Fv- oder "single chain Fv"-Fragmente), welche die gleiche Epitopspezifität wie der vollständige Antikörper aufweisen. Die Herstellung solcher Fragmente ist dem Fachmann bekannt. Vorzugsweise handelt es sich bei den erfindungsgemäßen Antikörper um monoklonale Antikörper. Die erfindungsgemäßen Antikörper können gemäß Standardverfahren hergestellt werden, wobei vorzugsweise β3-Endonexin-long oder ein synthetisches Fragment davon, vorzugsweise ein Fragment mit einer Aminosäuresequenz, die bei β3-Endonexin-short nicht vorhanden ist, als Immunogen dienen. Verfahren zur Gewinnung monoklonaler Antikörper sind dem Fachmann ebenfalls bekannt.

In einer besonders bevorzugten Ausführungsform ist der genannte monoklonale Antikörper ein aus einem Tier (z.B. Maus) stammender Antikörper, ein humanisierter Antikörper oder ein chimäre Antikörper oder ein Fragment davon. Chimäre, menschlichen Antikörpern ähnelnde oder humanisierte Antikörper besitzen eine herabgesetzte potentielle Antigenität, jedoch ist ihre Affinität gegenüber dem Ziel nicht herabgesetzt. Die Herstellung von chimären und humanisierten Antikörpern bzw. von den menschlichen Antikörpern ähnelnden Antikörpern wurde ausführlich beschrieben (siehe beispielsweise Queen et al., Proc. Natl. Acad. Sci. USA 86 (1989), 1 0029, und Verhoeyan et al., Science 239 (1988), 1534). Humanisierte Immunglobuline weisen variable Grundgerüstbereiche auf, die im wesentlichen von einem humanen Immunglobulin stammen (mit der Bezeichnung Akzeptor-Immunglobulin) und die Komplementarität der determinierenden Bereiche, die im wesentlichen von einem nicht-menschlichen Immunglobulin (z. B. von der Maus) stammen (mit der Bezeichnung Donor-Immunglobulin). Die (der) konstante(n) Bereich(e) stammt (stammen), falls vorhanden, auch im wesentlichen von einem menschlichen Immunglobulin. Bei der Verabreichung an menschliche Patienten bieten humanisierte (sowie die menschlichen) Antikörper eine Reihe von Vorteilen gegenüber Antikörpern von Mäusen oder anderen Spezies:
(a) das menschliche Immunsystem sollte das Grundgerüst oder den konstanten Bereich des humanisierten Antikörpers nicht als fremd erkennen und daher sollte die Antikörperantwort gegen einen solchen injizierten Antikörper geringer ausfallen als gegen einen vollständig fremden Maus-Antikörper oder einen partiell fremden chimären Antikörper,
(b) da der Effektorbereich des humanisierten Antikörpers menschlich ist, interagiert er besser mit anderen Teilen des menschlichen Immunsystems, und
(c) injizierte humanisierte Antikörper weisen eine Halbwertszeit auf, die im wesentlichen zu der von natürlich vorkommenden menschlichen Antikörpern äquivalent ist, was es erlaubt, kleinere und weniger häufige Dosen im Vergleich zu Antikörpern anderer Spezies zu verabreichen.

Der hier verwendete Ausdruck "β3-Endonexin-long" oder "ein eine β3-Endonexin-long kodierende DNA enthaltender Expressionsvektor" betrifft jede Form des Proteins selbst bzw. die von der DNA kodierte Form, die als ein dominant negativer Inhibitor der β3-A-Internalisierung wirken und dadurch B3-Integrin-Intemalisierungs-abhängige Prozesse bzw. die Sekretion von pro-atherogenen Mediatoren aus Thrombozyten hemmen kann. Dazu zählt nicht nur die Wildtypform, sondem auch Formen, die gegenüber der Wildtypform Veränderungen in der Aminosäuresequenz, wie Substitutionen von einer oder mehreren Aminosäuren, Deletionen oder Additionen, aufweisen, ohne dass dadurch die ursprüngliche biologische Aktivität wesentlich verändert wird.

Für die Insertion und Expression der vorstehend diskutierten β3-Endonexin-long kodierenden DNA, können ebenfalls eine Reihe von dem Fachmann bekannten Expressionsvektoren verwendet werden, wobei bezüglich dieser Expressionsvektoren die vorstehenden Ausführungen bezüglich der Forderung von β3-Integrin-Internalisierung-abhängigen Prozessen bzw. die Hemmung der Sekretion von pro-atherogenen Mediatoren aus Endothelzellen und die Hemmung der Expression von migrationsfördernden Oberfächenrezeptoren auf Endothelzellen sinngemäß gelten. Bezüglich der rekombinanten Herstellung von B3-Endonexin-long, geeigneten Expressionsvektoren, Wirtszellen, Züchtungs- und Reinigungsverfahren gelten ebenfalls die vorstehend bezüglich R3-Endonexin-short gemachten Ausführungen sinngemäß.

Der hier verwendete Ausdruck "NITY-Motiv enthaltendes Peptid oder Polypeptid" betrifft ein Peptid oder Polypeptid jeglicher Art, das das NITY-Motiv aufweist, und an β3-Endonexin-short binden und somit letzteres an der Bindung an β3-Integrin hindern kann. Das NITY-Motiv kann in Wildtypform, aber auch in Formen vorliegen, die gegenüber der Wildtypform Veränderungen in der Aminosäuresequenz, wie Substitutionen von einer oder mehreren Aminosäuren, Deletionen oder Additionen, aufweisen, ohne dass dadurch die Bindungsaffinitätzu β3-Endonexin-short wesentlich verändert ist.

Der hier verwendete Ausdruck "eine die Bindung von β3-Endonexin-short an das NITY-Motiv von β3-Integrin hemmende Verbindung" betrifft eine Verbindung jeglicher Art, z. B. eine synthetische oder natürlich vorkommende Verbindung, die solches vermag, z.B. dadurch, dass sie an das NITY-Motiv von β3-Integrin bindet, wodurch dieses nicht mehr für eine Bindung mit β3-Endonexin-short zur Verfugung steht. Ein Beispiel für eine solche Verbindung ist ein Antikörper, der das NITY-Motiv von β3-Integrin erkennt. Es wird auf die vorstehenden allgemeinen Ausführungen hinsichtlich Antikörper verwiesen. Allgemein können Verbindungen, die die Bindung von β3-Endonexin-short an das NITY-Motiv von β3-Integrin hemmen können, über verschiedene Verfahren identifiziert und bereitgestellt werden. Es wird auf die Beschreibung der nachstehenden Verfahren verwiesen.

Die vorliegende Erfindung erlaubt auch die Durchführung therapeutischer Maßnahmen bei Erkrankungen, die mit dysregulierten β3-Integrin-abhängigen intrazellulären Prozessen in Zusammenhang stehen, oder die einerseits durch eine Förderung von β3-Integrin-internalisierungsabhängigen Prozessen bzw. durch eine Hemmung von pro-atherogenen Mediatoren aus Endothelzellen und durch eine Hemmung der Expression von migrationsfördernden Oberflächenrezeptoren an Endothelzellen oder andererseits durch eine Hemmung von β3-Integrin-Internalisierungs-abhängigen Prozessen bzw. durch eine Hemmung der Sekretion von pro-atherogenen Mediatoren aus Thrombozyten therapiert werden können. Die vorstehend beschriebenen Verbindungen können zur Behandlung von Krankheitszuständen verwendet werden, die mit einer gesteigerten Aktivität von B3-Integrin-Internalisierungs-abhängigen Prozessen bzw. mit einer gesteigerten Sekretion von pro-atherogenen Mediatoren aus Thrombozyten assoziiert sind, oder die durch eine Hemmung von β3-Integrin-Intemalisierungsabhängigen Prozessen bzw. durch eine Hemmung der Sekretion von pro-atherogenen Mediatoren aus Thrombozyten therapiert werden können. Vorzugsweise handelt es sich bei diesen Erkrankungen um Syndrome, die mit einer verstärkten Sekretion vasoaktiven Mediatoren (Wachstumsfaktoren, Cytokine, Koagulationsfaktoren und andere) aus a-Granula von Thrombozyten oder Weibel-Palade bodies von Endotheizellen einhergehen.

Andererseits können die vorstehend beschriebenen Verbindungen auch zur Behandlung von Krankheitszuständen verwendet werden, die mit einer verringerten Aktivität von β3-Integrin-Internalisierungs-abhängigen Prozessen bzw. einer verringerten Sekretion von pro-atherogenen Mediatoren aus Endothelzellen und einer verringerten Expression von migrationsfordernden Oberflächenrezeptoren auf Endothelzellen assoziiert sind, oder die durch eine Forderung von β3-Integrin-Internalisierungs-abhängigen Prozessen bzw. einer Hemmung der Sekretion von pro-atherogenen Mediatoren aus Endothelzellen bzw. einer Hemmung der Expression von migrationsfördemden Oberflächenrezeptoren auf Endotheizellen therapiert werden können.

Vorzugsweise handelt es sich bei diesen Erkrankungen um akute und chronische Gefäßerkrankungen, wie Atherosklerose und instabile Gefäßplaques, akuter Herzinfarkt, Schlaganfall, peripher arterielle Verschlußkrankheit, chronisch venösen Ulcus der Extremitäten, Restenosen nach interventionellen Eingriffen und andere.

Die erfindungsgemäßen Arzneimittel enthalten vorzugsweise zusätzlich einen pharmazeutisch vertraglichen Träger. Geeignete Träger und die Formulierung derartiger Arzneimittel sind dem Fachmann bekannt. Zu geeigneten Trägem zählen beispielsweise Phosphat-gepufferte Kochsalzlösungen, Wasser, Emulsionen, beispielsweise Öl/Wasser-Emulsionen, Netzmittel, sterile Lösungen etc. Die Verabreichung der Arzneimittel kann oral oder parenteral erfolgen. Zu den Verfahren für die parenterale Verabreichung gehören die topische, intra-arterielle, intramuskuläre, subkutane, intra-medullare, intrathekale, intraventrikuläre, intravenöse, intraperitoneale oder intranasale Verabreichung. Die geeignete Dosierung wird von dem behandelnden Arzt bestimmt und hängt von verschiedenen Faktoren ab, beispielsweise von dem Alter, dem Geschlecht, dem Gewicht des Patienten, der Art und dem Stadium der Erkrankung, der Art der Verabreichung etc.

Ein weiterer Gegenstand der vorliegenden Erfindung ist ein Verfahren, mit dem Verbindungen identifiziert werden können, die eine Bindung von β3-Endonexin-short an das NITY-Motiv von β3-Integrin hemmen. Ein solches Verfahren umfaßt die Inkubation von β3-Endonexin-short mit β3-Integrin und den zu testenden Verbindungen sowie den Nachweis der Bindungshemmung von β53-Endonexin-short an β3-Integrin. Günstig kann es sein, wenn β3-Endonexin-short in Form eines das NITY-Motiv erkennenden Peptides oder Polypeptides vorliegt. Auch kann es günstig sein, wenn β3-Integrin in Form eines das NITY-Motiv umfassenden Peptids oder Polypeptids vorliegt. Ferner kann es günstig sein, wenn eine Kombination aus beiden Afternativen vorliegt. Des weiteren kann es günstig sein, wenn β3-Endonexin-short bzw. das das NITY-Motiv erkennende Peptid oder Polypeptid davon und/oder β3-Integrin bzw. das NITY-Motiv enthaltende Peptid oder Polypeptid in Form von sie kodierenden Express ionsvektoren vorliegen.

Besonders günstig ist ein Verfahren, bei dem β3-Endonexin nach Biotinylierung auf Avidin-Mikrotiterplatten immobilisiert und das NITY-Motiv in Form eines 10mer Peptides, wobei das Peptid mit Fluorescein gekoppelt ist, zusammen mit einer zu testenden Verbindung zugegeben wird. Durch Bestimmung der Fluoreszenz wird die Bindungshemmung der zu testenden Verbindung festgestellt.

Ferner ist ein Verfahren besonders günstig, bei dem β3-Endonexin-short fusioniert mit dem Green-Fluorescence-Protein (GFP), wie vorstehend beschrieben, auf Mikrotiterplatten immobilisiert und das vorstehende 10mer Peptid zusammen mit der zu testenden Verbindung zugegeben wird. Durch Bestimmung des Resonanzenergietransfers zwischen Fluorescein und GFP wird die Bindungshemmung der zu testenden Verbindung festgestellt.

Des weiteren ist ein Verfahren besonders günstig, bei dem Zellen, z.B. CHO-Zellen, verwendet werden, die GPIIb/IIIa und β3-Endonexin-short exprimieren. Nach Zugabe einer zu testenden Verbindung wird die Bindungshemmung der Verbindung mittels der GPIIb/IIIa-Internalisierung durch Messung Über Antikörperbindung und FACS bestimmt.

### Kurze Beschreibung der Figuren:

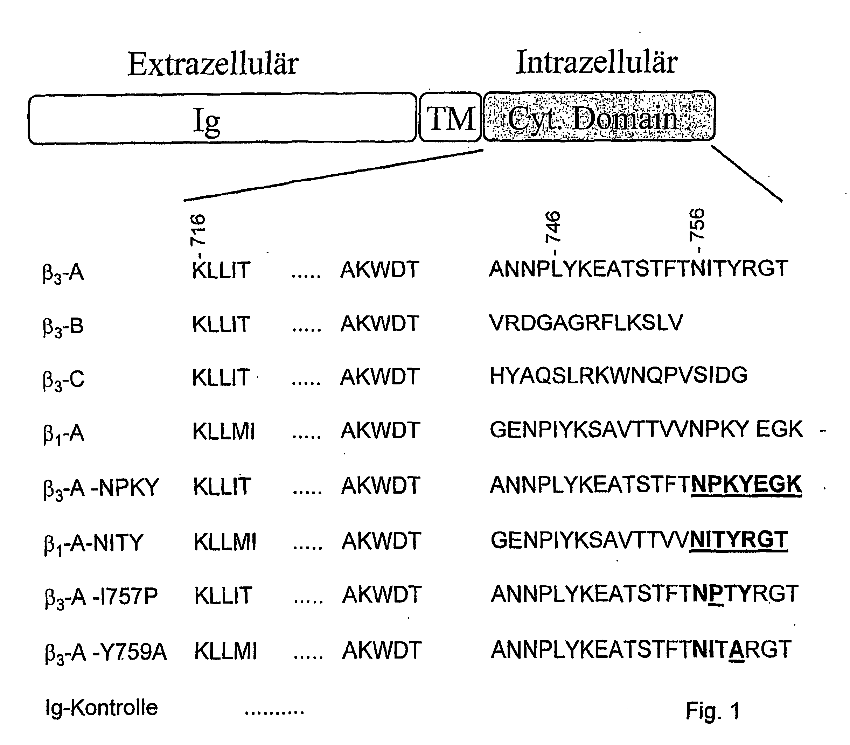

### Figur 1: Schematische Darstellung der verwendeten chimären Rezeptoren

Es sind die Aminosäuresequenzen der zytoplasmatischen Domänen von β1-A, Wildtyp-β3-B, alternativ gespleißten Formen von β3 und Mutanten-β3-A dargestellt. Intrazelluläre Domänen von β3-Integrinen (Wildtyp und Mutanten) wurden als chimäre Einzelketten-Rezeptoren exprimiert, die an Heterologe extrazelluläre Domänen und Transmembran-Domänen fusioniert worden waren, die den CH2- und CH3-Domänen von humanem IgG1 bzw. des CD7-Antigens entsprechen. Die Unterstreichungen markieren veränderte Aminosäuresequenzen.

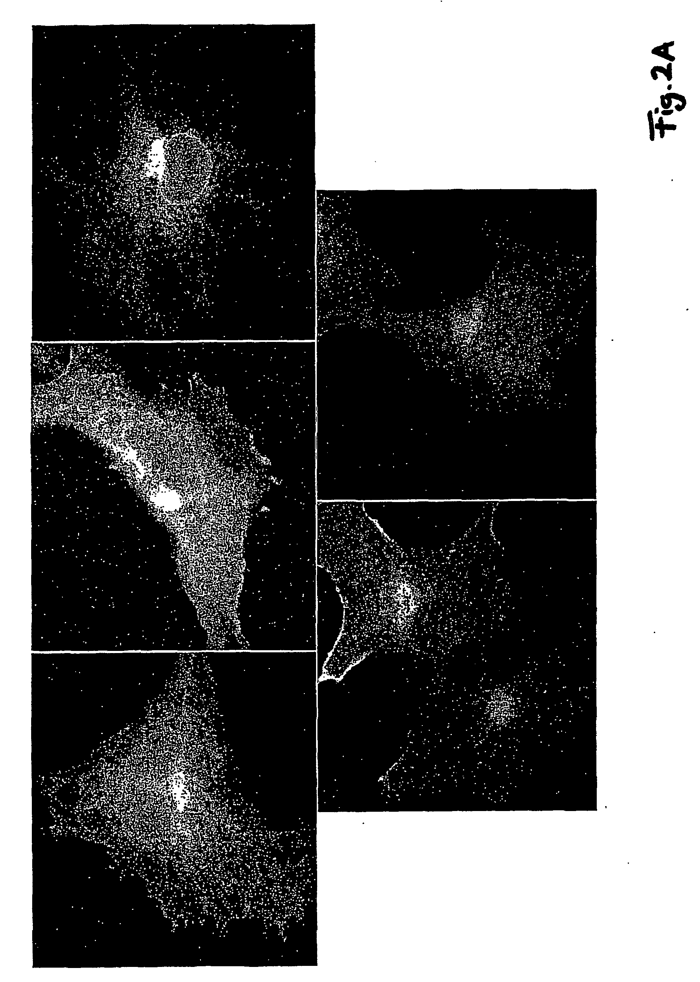

### Figur 2A: Doppel-Immunfluoreszenz-Mikroaufnahme von mit β3-Isoform-Fusionsproteinen transient transfizierten CHO-Zellen: Die Oberflächenexpression von Ig-β3-A ist wesentlich verringert

CHO-Zellen wurden auf Deckgläschen plattiert und bis zur Subkonfluenz wachsen gelassen. Danach wurden die Zellen mit Aliquots von Plasmid-DNAs transfiziert, die für verschiedene Ig-β3-Integrin-Chimären (β3-A, β3-B, β3-C, β1-A) bzw. Ig-Kontrollkonstrukte kodieren. Nach 24 Stunden wurden die Zellen fixiert und die Zell-Oberflächenexpression der Fusionsproteine wurde durch Anfärbung mit einem TxR-konjugierten Maus-anti-IgG1-Antikörper (rote Immunfluoreszenz) nachgewiesen. Danach wurden die Zellen mit 0,02% Triton X-100™ permeabilisiert und die intrazelluläre Expression der Chimären wurde durch Anfärbung mit einem FITC-konjugierten Maus-anti-IgG1-Antikörper (grüne Fluoreszenz) nachgewiesen. Es ist zu beachten, dass alle Chimären innerhalb der Zellen diffus exprimiert werden (grün). Bezeichnenderweise können alle Chimären außer Ig-β3 auf der Zelloberfläche nachgewiesen werden (rot).

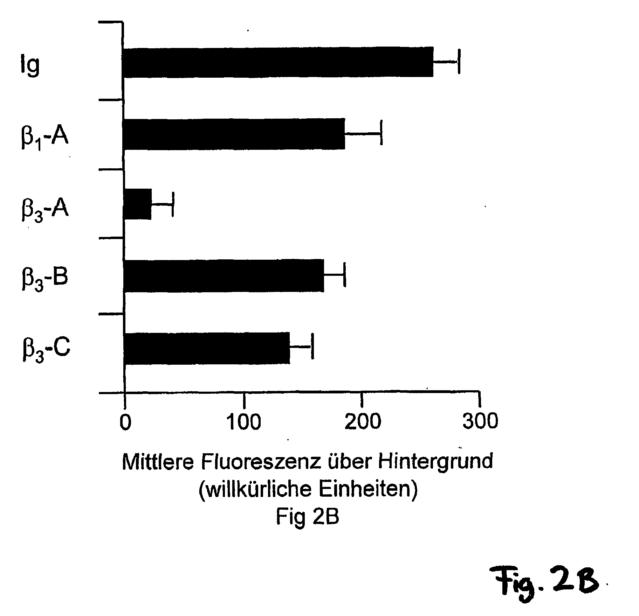

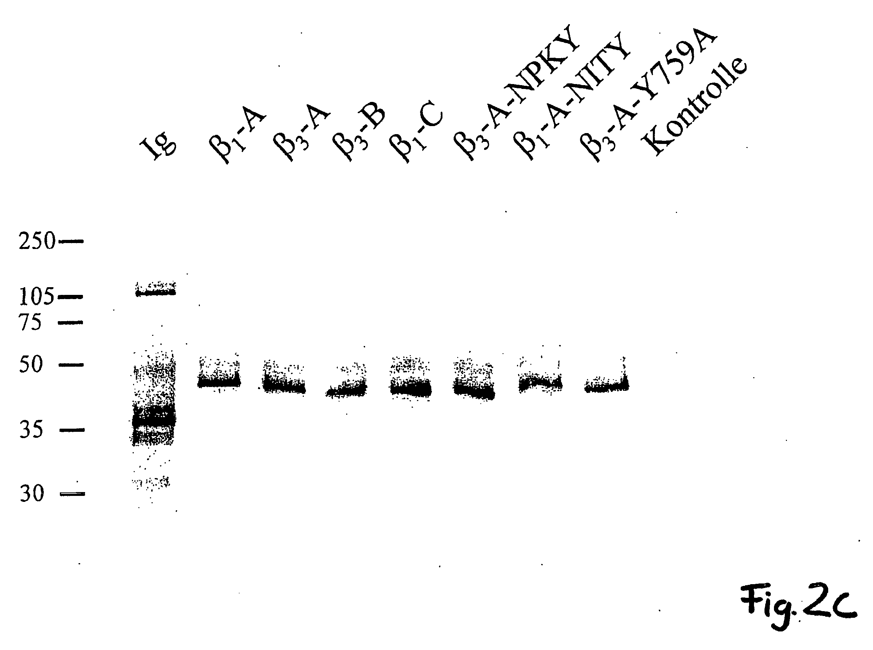

### Figur 2B: Durchflußzytometrie-Analyse der Zelloberflächenexpression von β3-Isoform-Chimären in CHO-Zellen

Subkonfluente Monolayer von CHO-Zellen wurden mit chimären Konstrukten der β3-Isoformen (Ig-β3-A, Ig-β3-B, Ig-β3-C) transient transfiziert. Als Kontrollen wurden die Ig-β1-A-Chimären oder das schwanziose Ig-Kontrollkonstrukt verwendet. Die Zellen wurden mit 2% Formaldehyd fixiert und mit einem TxR-konjugierten Maus-anti-IgG1-Antikörper angefärbt. Danach wurden die Zellen gewaschen und in 0,02% Triton X-10TM und Sättigungskonzentrationen an FITC-konjugiertem Maus-anti-IgG1-Antikörper enthaltender PBS zum Nachweis der intrazellulären Expression der entsprechenden Chimären resuspendiert. Proben wurden anschließend über Durchflußzytometrie analysiert. Gezeigt sind die Mittelwerte der Ergebnisse von drei unabhängig durchgeführten Experimenten. Hintergrund-Immunfluoreszenz-Spiegel wurden mittels "Mock"-Transfektanten (Vektor-DNA) definiert.

### Figur 2C: Expression von β1- und β3-Isoform-Chimären

Subkonfluente Lagen von COS-Zellen wurden mit den angegebenen chimären Konstrukten transient transfiziert. Nach 24 Stunden wurden die Zellen lysiert und die Ig-Fusionsproteine an Protein A-Sepharose präzipitiert. Danach wurde ein Immunblot-Analyse mittels eines anti-Ig-Antikörpers durchgeführt,

### Figur 3A: Zwei-Farben-Immunofluoreszenz-Mikroaufnahme von CHO-Zellen, die Fusionsproteine von β3-Integrin-NITY-Motiv-Mutanten oder Varianten transient exprimieren: Das zytoplasmatische NITY-Motiv tragende Chimären sind innerhalb der Zelle lokalisiert

CHO-Zellen wurden auf mit Vitronectin vorbeschichteten Deckgläschen ausplattiert und mit den β3-Integrin-Mutanten β1-A-NITY, β3-A-NPKY, β3-A-1757P, β3-A-Y759A, den β3-A- und β1-A-Isoformen oder dem schwanzlosen Ig-Kontrollvektor transient transfiziert. Nach 24 Stunden wurden die Zellen fixiert und wie in der Legende zu Figur 2 beschrieben gefärbt. Die rote Immunfluoreszenz deutet auf Oberflächenexpression hin, die grüne auf intrazellulare Expression der chimären Konstrukte. Es ist zu beachten, dass alle Chimären gleich und diffus innerhalb der Zelle exprimiert werden (grün) und dass Punktmutationen oder Deletionen des NITY-Motivs zur einer erhöhten Zelloberflächenexpression führen.

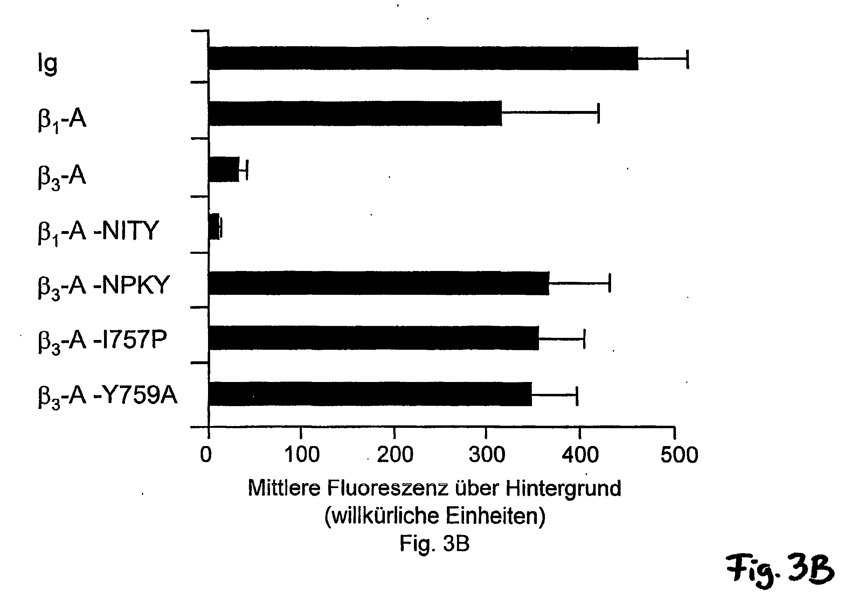

### Figur 3B: Quantifizierung der Zelloberflächenexpression von NITY-Motiv-Mutanten

CHO-Zellen wurden mit chimären Konstrukten der β3-NITY-Mutanten transient transfiziert. Die Zellen wurden gefärbt und die Zelloberflächenexpression der Chimären wurde wie in der Legende zu Figur 2 beschrieben analysiert. Gezeigt sind die Mittelwerte der Ergebnisse von drei unabhängig durchgeführten Experimenten.

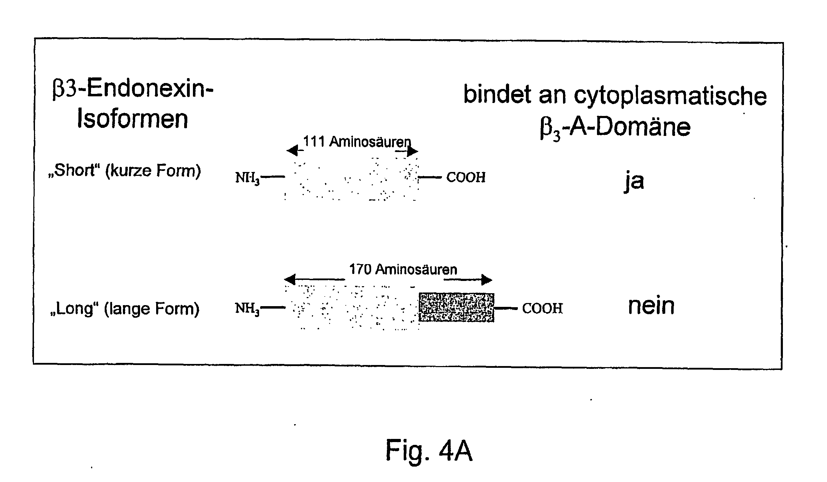

### Figur 4A: Schematische Darstellung der β3-Endonexin-Isoformen

### Figur 4B: Westernblot-Analyse von GFP-Endonexin-Fusionsproteinen in CHO-Zellen

Aliquots von Gesamtlysaten von CHO-Zellen, die mit den angegebenen Konstrukten transfiziert waren, wurden über SDS-PAGE aufgetrennt, und mittels eines anti-GFP-Antikörpers wurde eine Immunblot-Analyse durchgeführt.

### Figur 4C: Zellobeflächenexpression von β3-Isoform-Ig-Fusionsproteinen (rot) in CHO-Zellen, die mit GFP-Fusionsproteinen von β3-Endonexin-Isoformen cotransfiziert waren (grün): GFP-En-L bzw. GFP-En-L-ΔK⁶²RKK vermitteln spezifisch die Zelloberflächenexpression einer Ig-β3-A-Chimären, jedoch nicht von Ig-β3-B- oder Ig-β3-C-Chimären

CHO-Zellen wurden mit β3-Integrin und GFP/β3-Endonexin-Isoformen transienl cotransfiziert. Nach 24 Stunden wurden die Zellen fixiert und hinsichtlich der Zelloberflächenexpression von β3-Chimären mit einem TxR-konjugierten Maus-anti-IgG₁-Antikörper (rote Immunfluoreszenz) abgefärbt. Es ist zu beachten, dass Ig-β3-A auf der Zelloberfläche von Zellen exprimiert wird, die mit der langen Form van β3-Endonexin cotransfiziert sind (obere Reihe, drittes Bild von links).

### Figur 4D: Quantifizierung der Zelloberflächenexpression von Chimären der zytoplasmatischen Domäne von β3-Integrin in mit β3-Endonexin-Isoformen cotransfizierten CHO-Zellen

CHO-Zellen wurden mit Ig-β3-A-, Ig-β1-A-, Ig-β1-NITY- oder Ig-β3-NPKY-Expressionskonstrukten zusammen mit GFP/β3-Endonexin-Isoformen transient transfiziert. Nach 24 Stunden wurden die Zellen fixiert und hinsichtlich der Zelloberflächenexpression der Fusionsproteine mit TxR-konjugiertem Maus-anti-IgG₁ (rote Immunfluoreszenz) abgefärbt. 10.000 grüne (GFP)-positive Zellen wurden beurteilt. Gezeigt sind die Mittelwerte der Ergebnisse von drei unabhängig durchgeführten Experimenten.

### Figur 5A: Ein exogener anti-Ig-Antikörper wird von Ig-β3-A exprimierenden Zellen schnell internalisiert

CHO-Zellen wurden auf mit Vitronectin vorbeschichteten Deckgläschen ausplattiert und mit Ig-β3-A, Ig-β3-B, Ig-β3-C, Ig-β1-A oder den schwanzlosen Ig-Kontrollkonstrukten transient transfiziert. Nach 24 Stunden wurden 5 µl/ml TxR-konjugierter anti-IgG1-MAK zu dem Kulturmedium gegeben und es wurde 15 Min. inkubiert. Die Internalisierung wurde durch die Zugabe von eiskaltem Fixierungsmittel zu den Zellen zu den angegebenen Zeitabschnitten beendet. Danach wurden die Zellen mittels Immunfluoreszenz-Mikroskopie beurteilt. Es ist zu beachten, dass im Gegensatz zu den anderen Chimären β3-A-transfizierte Zellen keine wesentliche Zelloberflächenfärbung zeigen. Die grüne Immunfluoreszenz akkumuliert jedoch innerhalb von Minuten in intrazellulären vesikulären Kompartimenten.

### Figur 5B: β3-Endonexin-L-Konstrukte blockieren die konstitutive Internalisierung von Ig-β3-A

CHO-Zellen wurden auf mit Vitronectin vorbeschichteten Deckgläschen ausplattiert und mit den β3-Integrin-β3-A- und β3-Endonexin-Isoformen transient cotransfiziert. Nach 24 Stunden wurden 5 µl/ml TxR-konjugierter anti-IgG₁-MAK zu dem Kulturmedium gegeben und 15 min inkubiert. Die Internalisierung wurde durch die Zugabe von eiskaltem Fixierungsmittel zu den Zellen zu den angegebenen Zeitabschnitten beendet. Danach wurden die grün fluoreszierenden Zellen mittels Zwei-Farbenlmmunfluoreszenz-Mikroskopie beurteilt. Es ist zu beachten, dass in mit En-L-Konstrukten transfizierten Zellen die Internalisierung von β3-A wesentlich reduziert ist.

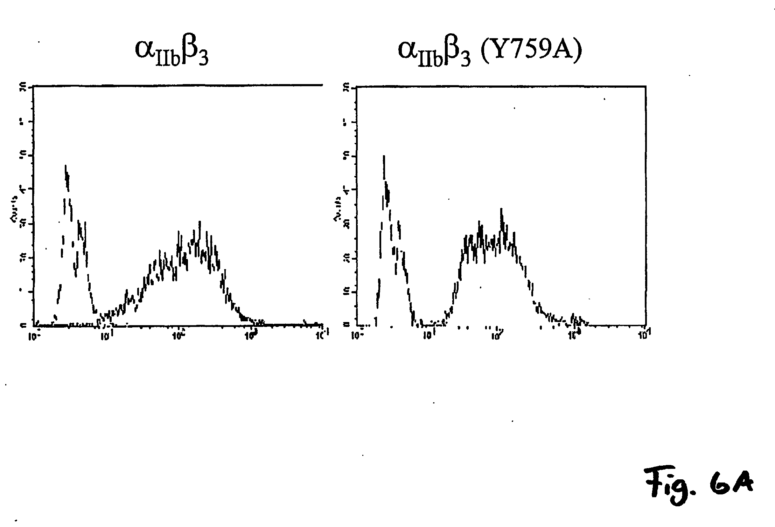

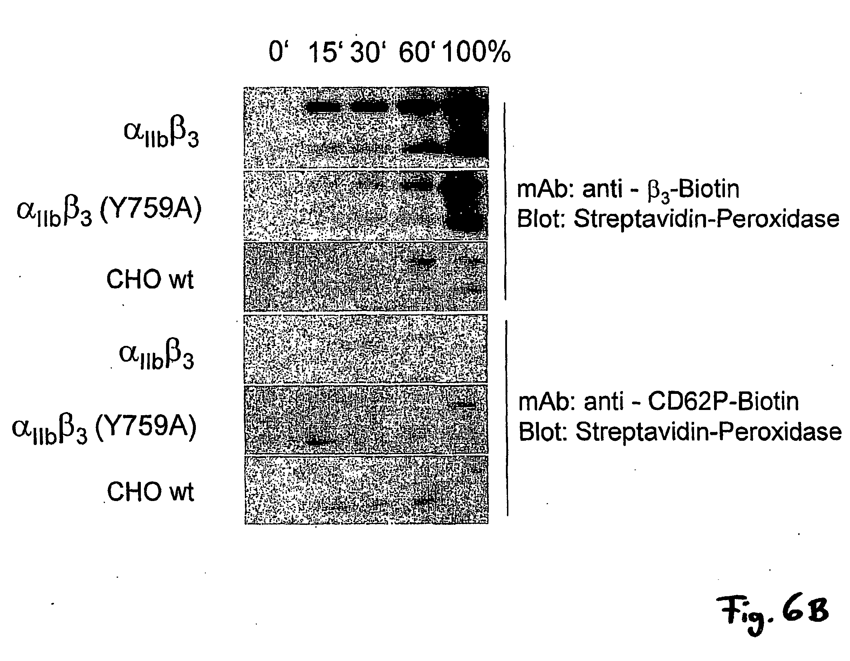

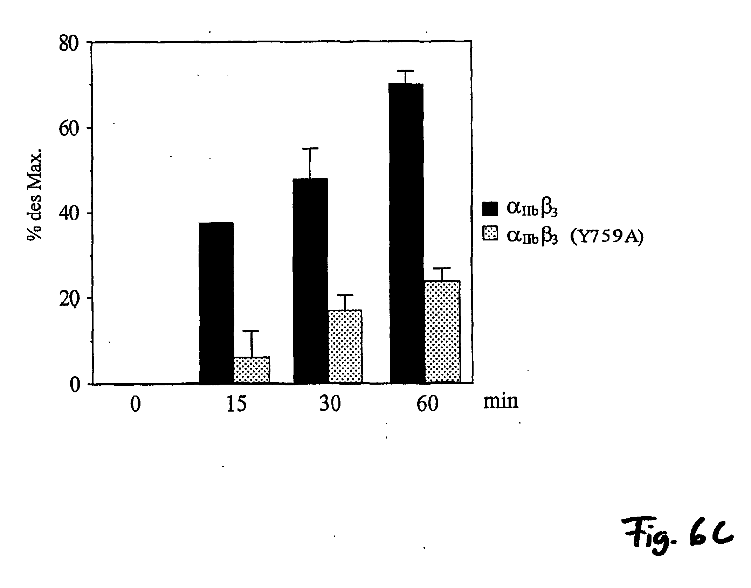

### Figur 6: Messung der Endozytose von nativem β3-Integrin: biochemische Beurteilung

(A) Durchflusszytometrie-Analyse von stabilen α_{IIb} mit Wildtyp-β3 oder die β3-Mutante Y759A exprimierenden Zellinien. Es sind die Fluoreszenz-Histogramme mit negativem Kontroll-Antikörper (anti-CD62, links) oder anti-β3 (mAb15) (rechts) konjugiert mit Fluorescein-Isothiocyanat gezeigt.
(B) CHO-Zellen wurden 15 min mit Biotin-markiertem anti-β3-MAK auf Eis vorinkubiert und danach bei 37°C zu den angegebenen Zeiträumen inkubiert. Nach Inkubation mit dem Reduktionsmittel wurden die Zellen lysiert, und die Proteine über SDS-PAGE aufgetrennt und mittels Immunblots unter Verwendung von Peroxidase-gekoppeltem Streptavidin nachgewiesen. Für die halbquantitative Beurteilung der Fraktion Biotinmarkierter MAKs, die von dem Reduktionsmittel geschützt wurden, wurden Aliquots von Parallelproben nach kalter Inkubation entnommen und zur Erleichterung der Abschätzung des internalisierten Materials als Fraktion des Gesamtinputs als Normalisierungskontrollen beladen (rechte Spuren, 100, 50, 10%, α_{IIb}β3, bzw. 80, 40, 20%, α_{IIb} β3 (Y759A)). Alle Proben wurden als Duplikate entnommen.
(C) Entsprechende Ergebnisse des Dichte-Scanning der Antikörper-Banden (OD=optische Dichte, beliebige Einheiten)

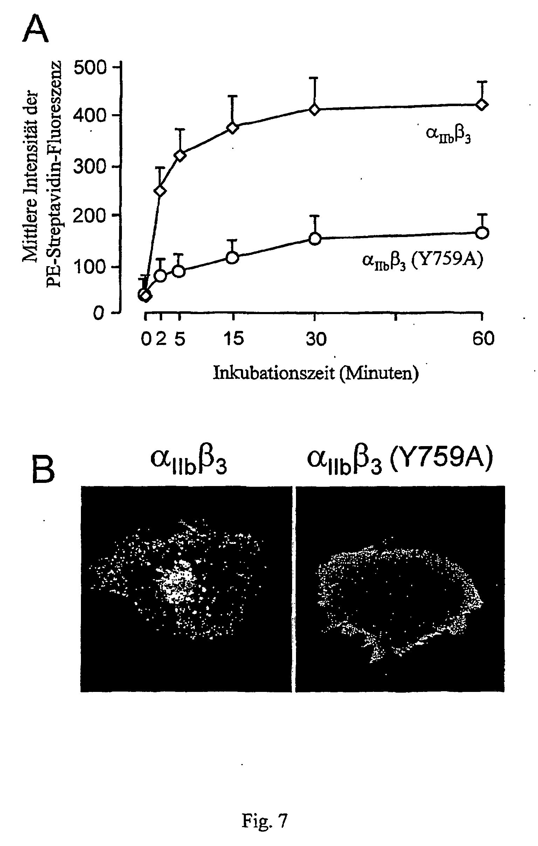

### Figur 7: Messung der Endozytose von nativem β3-Integrin; Immunfluoreszenz

(A) Quantitative Beurteilung der Internalisierung von Biotin-markiertem anti-β3-MAK in Wildtyp- α_{IIb}β3 und α_{IIb}β3 (Y759A) exprimierenden CHO-Zellen. Die Zellen wurden 15 Min. auf Eis mit einem Biotin-markierten anti-β3-MAK (5 µg/ml) inkubiert, danach mit Kulturmedium verdünnt und zu den angegebenen Zeiträumen bei 37°C inkubiert. Es folgten Waschschritte mit Dulbecco-modifizierter PBS und drei aufeinanderfolgende Inkubationen mit einer Lösung, die 50 mM 2-Mercaptoethansulfonsäure (Sigma), 10 mM NaCl, 1 mM EDTA, 50 mM Tris und 0,2% Rinderserumalbumin, pH-Wert 8,6, enthielt. Die Zellen wurden danach mit 2% Formaldehyd fixiert, mit 0,2% Triton X-100™ permeabilisiert, mit FITC-konjugiertem Streptavidin angefärbt und über Durchflusszytometrie analysiert.
(B) Qualitativer Vergleich der Aufnahme von Wildtyp-α_{IIb}β3 und α_{IIb}β3 (Y759A), die Zellen wurden wie vorstehend beschrieben behandelt. Es ist eine typische Zelle der 15 Min.-Probe gezeigt. Die Analysen wurden mittels eines konfokalen Laser-Scanning-Mikroskops (Leica) durchgeführt.

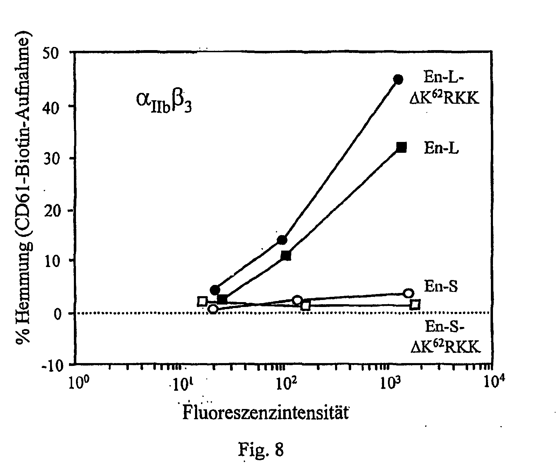

### Figur 8: Dosis-abhängige Analyse der Endozytose von nativem Wildtyo-β3-Integrin in transient mit β3-Endonexin-Isoformen transfizierten Zellen

Wildtyp-α_{IIb}β3 exprimierende CHO-Zellen wurden mit GFP/β3-Endonexin-Konstrukten oder GFP-Kontrollvektoren 24 Stunden transient transfiziert. Die Zellen wurden mit Biotin-markiertem anti-β3 15 Min. bei 4°C vorinkubiert, danach wurde weitere 15 min bei 37°C inkubiert. Nach Reduktion mit 2-Mercaptoethansulfonsäure wurden die Zellen mit PE-Streptavidin angefärbt, und etwa 10⁸ GFP-positive Zellen wurden mittels Durchflusszytometrie untersucht. Die mittlere Intensität an PE-Streptavidin wurde als Index der β3-Integrin-Endozytose verwendet und für verschiedene Niveaus des mittleren Expressionsspiegels von GFP relativ zu dem GFP-Kontrollvektor bestimmt. Dies wurde durch Analyse der Rot-Fluoreszenz innerhalb verschiedener "PMT" *Fluoreszenz-*Tore für verschiedene Niveaus der GFP-Fluoreszenz (grün) durchgeführt. Die Ergebnisse eines Experiments sind gezeigt; bei drei unabhängigen Experimenten wurden vergleichbare Ergebnisse beobachtet.

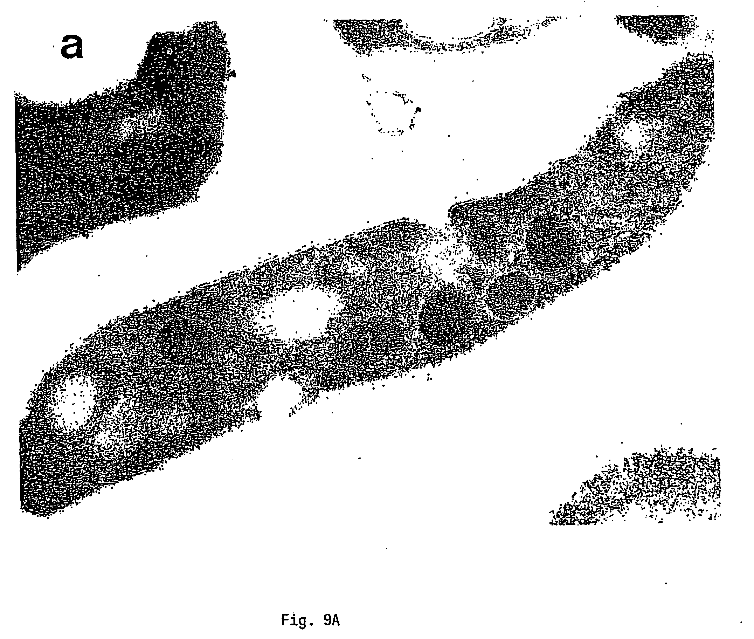

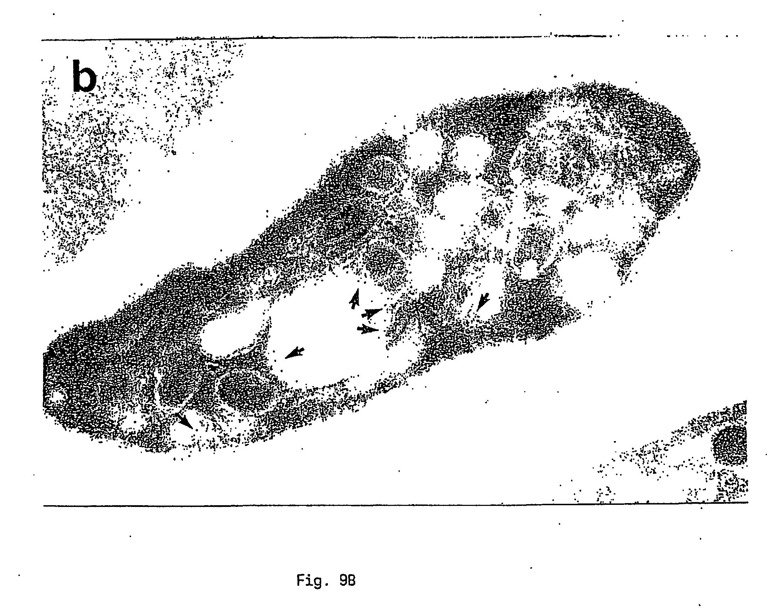

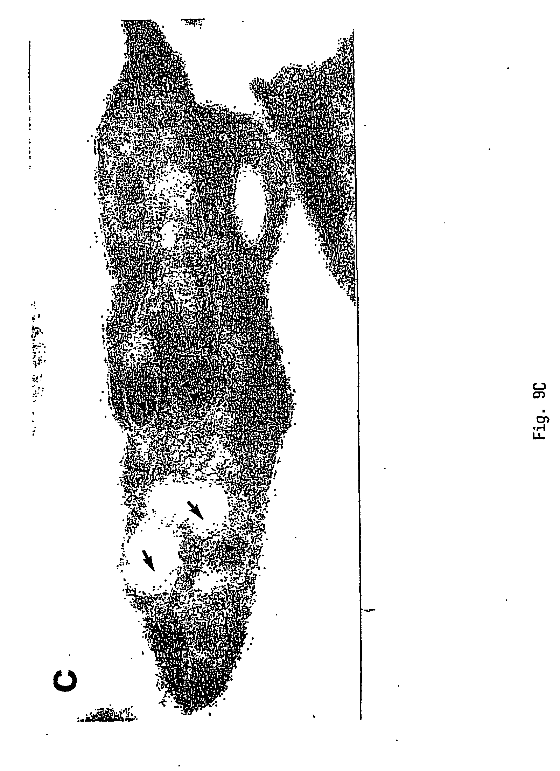

### Figur 9: Zeitabhängige β3-Integrin-Internalisierung in Speichervesikel (α-Granula) von Thrombozyten aus Patienten mit koronarer Herzerkrankung

Elektronenmikroskopische Darstellung von gewaschenen Blutplättchen (Thrombozyten) von Patienten, die wegen akuten Koronarsyndroms behandelt wurden. Die Thrombozyten wurden mit agonistischen Antikörpern gegen das β3-Integrin Glykoprotein IIb/IIIa (7E3-Antikörper), dann mit Gold-mankierten Anti-Maus Fab-Fragmenten 10 Minuten (A), 20 Minuten (B) oder 40 Minuten (C) bei Raumtemperatur inkubiert. Nach 10 Minuten befand sich der Immun-markierte 7E3-Antikörper (Pfeile) ausschließlich auf der Thrombozyten-Oberfläche. Nach 20 Minuten war die Oberflächen-Markierung vermindert; zunehmend befand sich markierter 7E3 im Oberflächen-verbundenen Membransystem ("SCS") der α-Granula. Nach 40 Minuten demarkiert sich der meiste GP IIb/IIIaindizierende 7E3-Antikörper in den α-Granula.

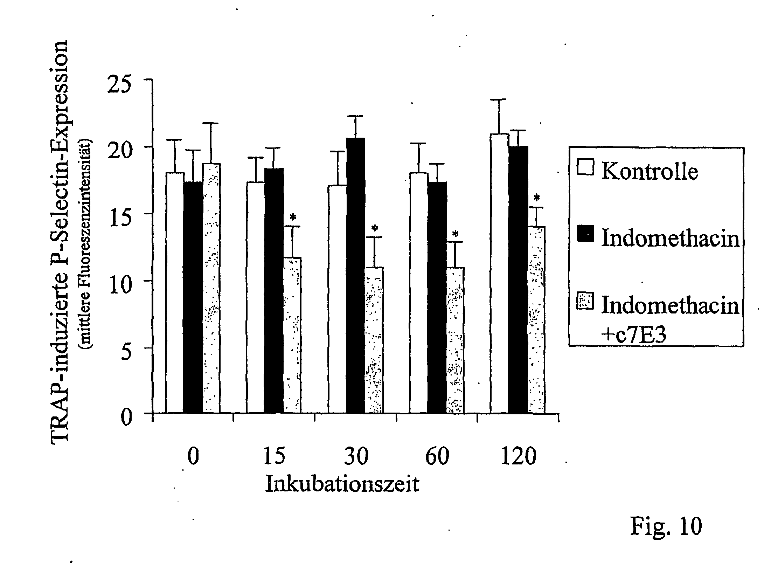

### Figur 10: Integrin-Internalisierung in α-Granula bewirkt verminderte α-Degranulation, gemessen als TRAP-induzierte P-Selektin-Obertlächenexpression auf menschlichen Thrombozyten

Effekt des Glykoprotein-IIb/IIIa-antagonistischen Abciximab auf die P-Selektin-Oberflächenexpression in TRAP (Thrombinrezeptor-aktivierendes Peptid) aktivierten Thrombozyten. Nach intravenöser Gabe von Abciximab (Eli-Lilly, Deutschland) wurde Thrombozyten-reiches Blutplasma von Patienten, die wegen akutem Koronarsyndrom behandelt wurden, zu den angegebenen Zeitpunkten gewonnen, und die TRAP (Thrombinrezeptor-aktivierendes Peptid; 25 µM)-induzierte P-Selektinexpression bestimmt. Der verwendete Antikörper CD62P erkennt dabei jenes Oberflächen-Selektin, das nach Degranulation dort exprimiert wird. Danach wurde das isolierte Thrombozyten-reiche Plasma in vitro mit Indomethacin (60 µM) allein oder Indomethacin und 7E3Antikorpern (50 µg/ml) inkubiert. Dann wurde die P-Selektin-Oberflächenexpression durchflusszytometrisch bestimmt.
* zeigt signifikante Änderung gegenüber dem Ausgangswert an (p< 0.05).

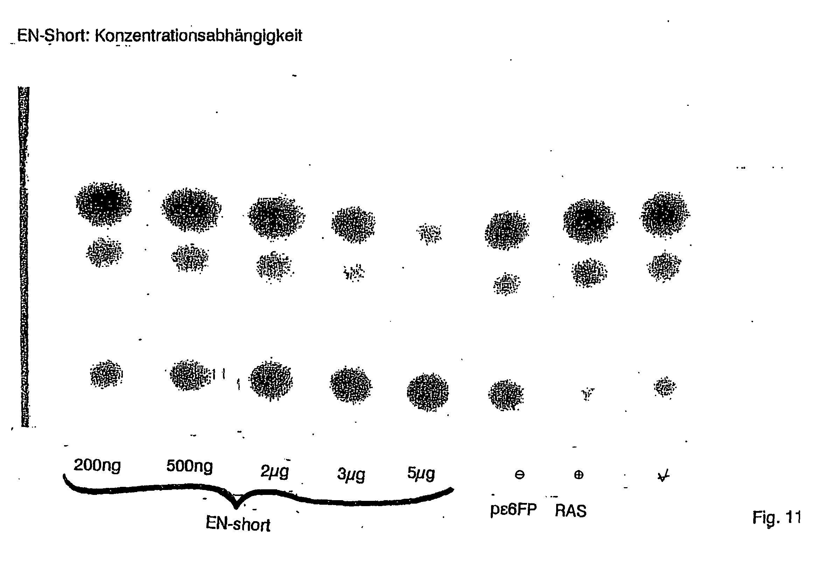

### Figur 11: β3-Endonexin-short reguliert den uPAR-Promotor herunter

CHO-Zellen wurden transient mit dem CAT-Reportergen transfiziert, das unter der Kontrolle des Urokinase-type Plasminogen Activator Receptor- (uPAR)-Promotors stand. Zusätzlich wurden verschiedene Mengen von β3-Endonexin-short-Plasmid (200 ng bis 5 µg; Spur 1 -5), dem leeren Expressionsvektor (pEGFP, Spur 6) oder leeren Liposomen (Spur 8) kotransfiziert. Zellextrakte, die hinsichtlich der Luziferase-Aktivität normalisiert worden waren, wurden mit ¹⁴C-Chloramphenicol inkubiert, mit Ethylacetat extrahiert und einer Dunnschichtchromatographie unterzogen. Die Umwandlung von ¹⁴C-Chloramphenicol zu acetylierten Derivaten wurde mittels eines "Phosphor-imager" durchgeführt. Die Ergebnisse sind in Figur 11 dargestellt.

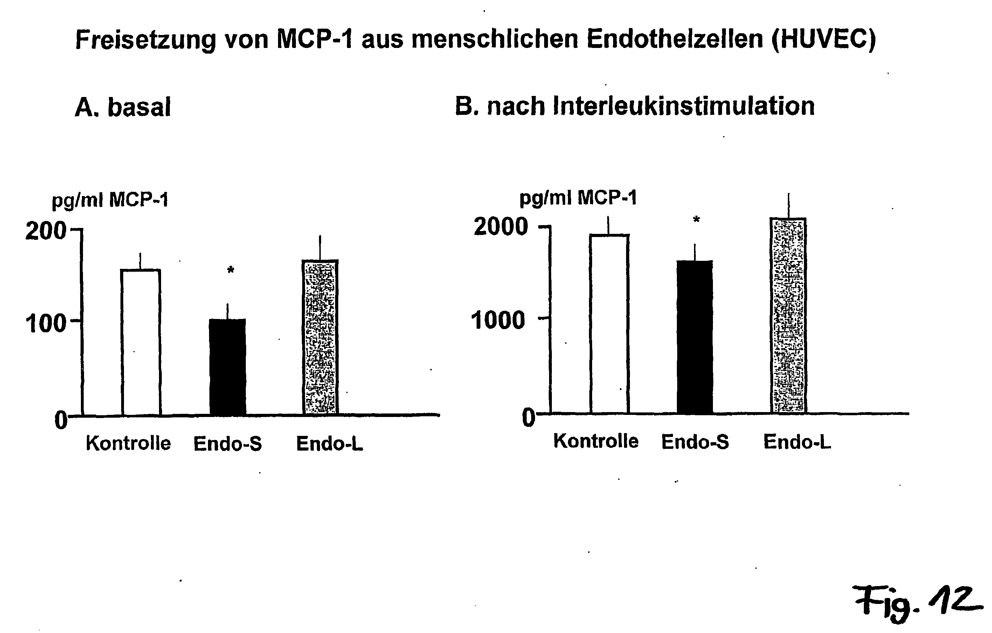

### Figur 12: Auswirkung der Expression von β3-Endonexin-short und β3-Endonexin-long auf die Sezernierung von MCP-1 von endothelialen Zellen

Kultivierte HUVEC-Zellen wurden entweder mit einem β3-Endonexin-short oder β3-Endonexin-long-kodierenden Plasmid-Konstrukt wie unter Beispiel 5 und 6 oder einem leeren Kontrollvektor transfiziert. 24 Stunden später wurden die Zellen entweder mit Vehikel oder 100 pg/ml Interleukin-1β über 120 Minuten inkubiert. Dann wurde der Überstand abgesaugt, und die Konzentrationen von Monozyten-chemotaktischem Protein 1 (MCP-1) wurden mittels ELISA bestimmt. Die Balken repräsentieren Ergebnisse von vier unabhängigen Experimenten mit der Angabe der mittleren Standardabweichung,

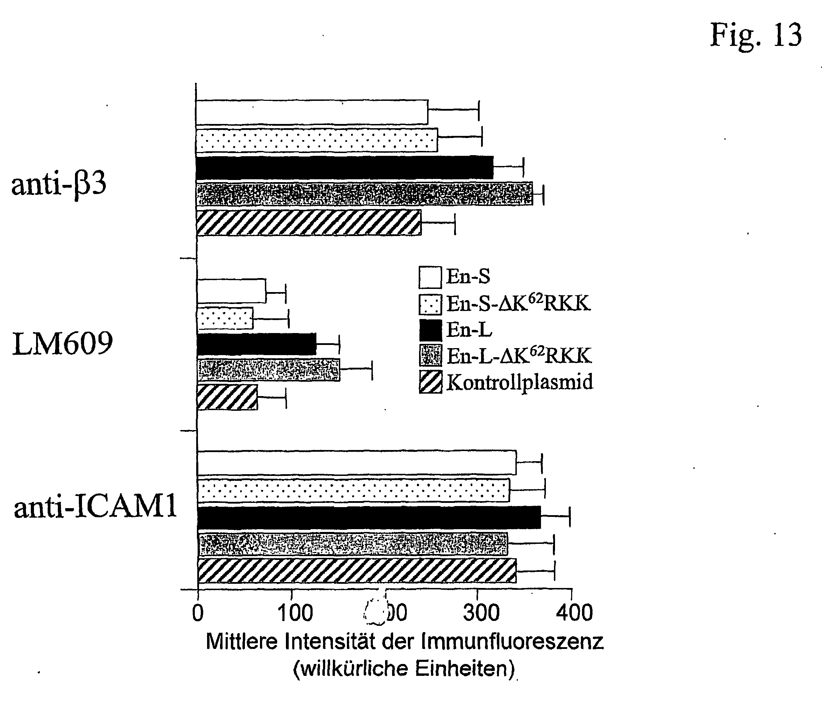

### Figur 13: Überexpression von Endonexin-long erhöht die Dichte von Vitronektinrezeptoren (VNR) auf menschlichen Endothelzellen (durchflusszytometrische Analyse)

Frisch isolierte humane umbilikale Endothelzellen (HUVEC-Zellen) wurden mit Plasmiden transfiziert, die für beide β3-Endonexine oder deren riukleäre Importmutanten kodierten. Die Zellen wurden geerntet, wie beschrieben fixiert und mit Antikörpern gefärbt. Dabei wurde der gegen β3-Integrine gerichtete Antikörper "anti-β3" und der gegen der Vitronektinrezeptor (VNR)-Komplex gerichtete Antikörper LM 609 verwendet. Als Kontrolle diente ein gegen den Adhäsionsrezeptor ICAM-1 gerichteter Antikörper. Gezeigt sind Mittelwerte von je 3 unabhängigen Versuchen. Die Ergebnisse beider gegen den VNR gerichteten Antikörper zeigen übereinstimmend, dass die Dichte der VNR nach Überexpression von En-L deutlich zunahm.

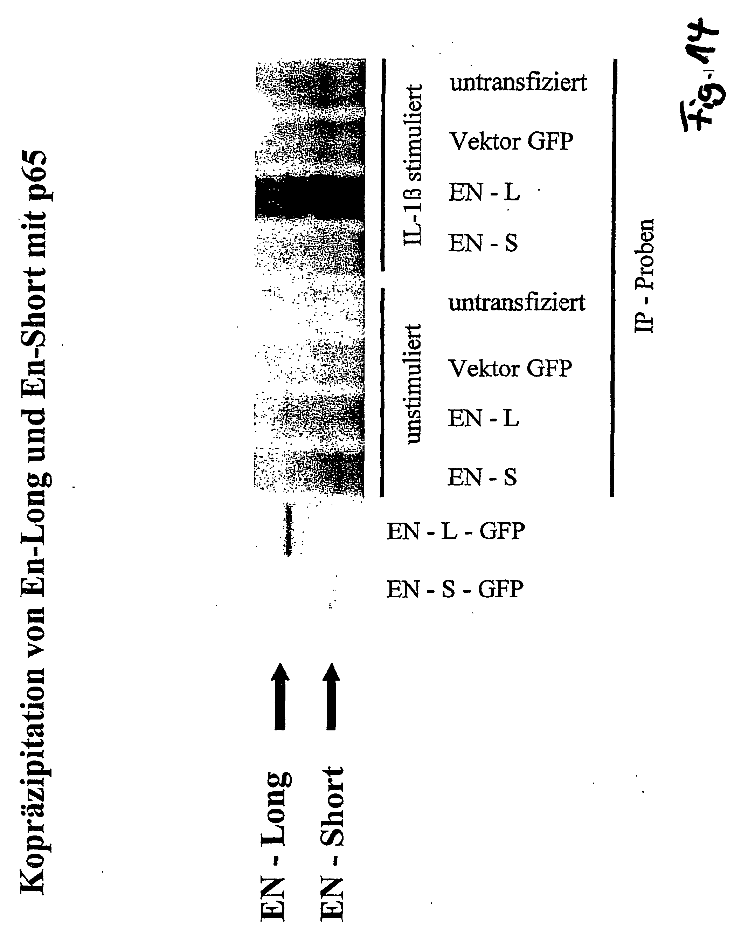

### Figur 14: Immunpräzipitationen von HUVEC-Zellen zeigen eine Co-Präzipitation von β3-Endonexin-short und β3-Endonexin-long mit der p65-Untereinheit von NF-kB.

HUVEC wurden mit rekombinantem En-S oder En-L transfiziert. 24 Stunden später wurden Extrakte aus diesen Zellen, die unter Basalbedingungen untersucht wurden oder mit Interleukin-1β stimuliert waren, wie beschrieben lysiert und gereinigt. Danach wurde mit Antikörpern gegen NF-kB (Biotin-konjugierter Maus anti-p65 mAb, Roche Biochemicals, Mannheim) immunpräzipitiert. Diese Präzipitate wurden auf SDS-Polyacrylamid-Gelen aufgetragen, auf Nitrozellulose überführt und mit den beschriebenen Antikörpern gegen β3-Endonexin geblottet. Die Ergebnisse zeigen, dass vor allem nach Zytokinstimulation En-S und En-L direkt an p65 von NF-kB binden und mit diesem interagieren.

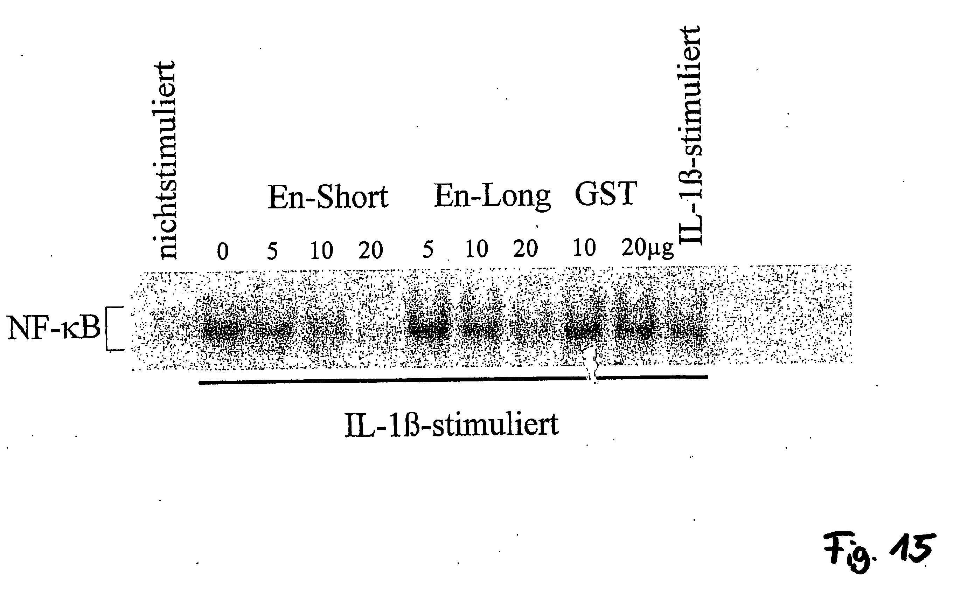

### Figur 15: Gel-Shift-Assays zur Messung der NF-kB-Aktivität zeigen eine Aufhebung der Aktivierbarkeit durch verschiedener Zytokine in Gegenwart von rekombinantem β3-Endonexin-short und β3-Endonexin-long:

Kernextrakte wurden aus IL-1β-stimutierten HUVEC präpariert und wie beschrieben analysiert. Die NF-kB-Bindung wird durch Überexpression von En-S in HUVEC aufgehoben und durch En-L deutlich vermindert. Als Negativkontrolle diente das zur Reinigung verwendete Protein GST. Die Ergebnisse zeigen, dass die NF-kB-Bindungsaktivität in Gegenwart steigender Dosen an En-S und En-L deutlich abnimmt. Damit wird auch die funktionelle Bedeutung der Interaktion von En-S und En-L mit p65 deutlich.

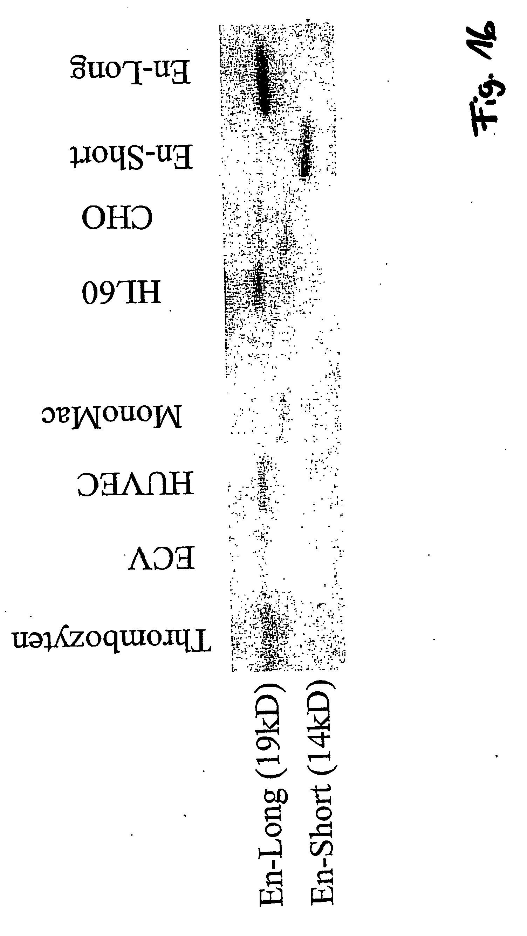

### Figur 16: β3-Endonexin-long, nicht β3-Endonexin-short, ist das dominante Protein in menschlichen Thrombozyten und Endothelzellen:

Der Blot zeigt Extrakte von menschlichen Thrombozyten und Endothelzellen, die mit dem spezifischen Antikörper gegen Endonexin geblottet wurden. Verwendet wurde die immortalisierte Endothelzellinie ECV und primärkultivierte umbilikale Endotheizellen (HUVEC). Als Kontolle dienten weitere Zellinien ("HL-60", "CHO") und gereinigte rekombinante Proteine (rechte Spuren). Erkennbar ist eine klare Bande auf Höhe von β3-Endonexin-long, während kein endogenes β3-Endonexin-short abgrenzbar ist. Dieser Befund zeigt, dass in menschlichen Thrombozyten und Endothelzellen En-L das dominante Protein ist.

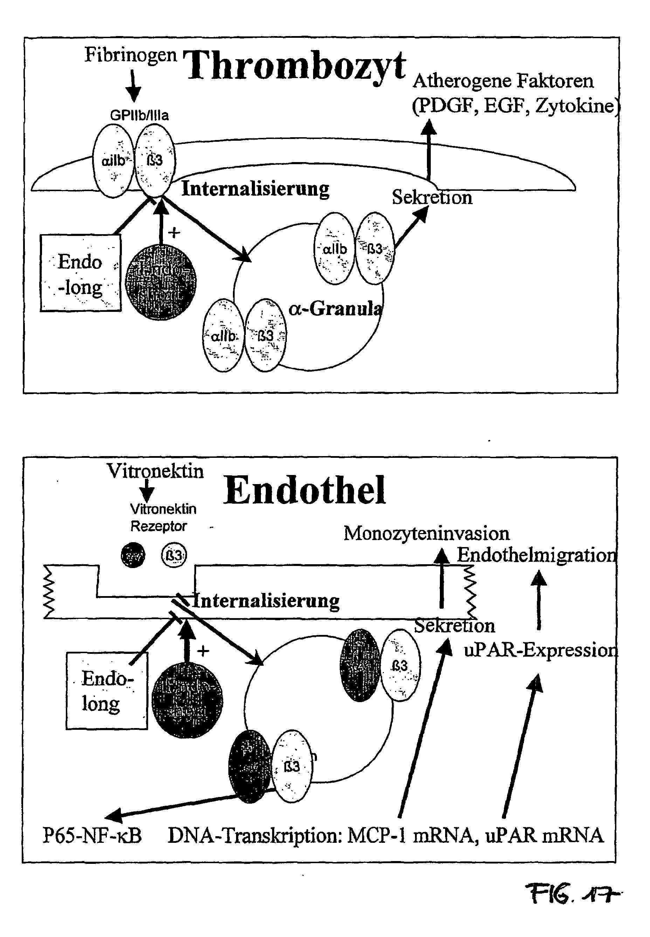

### Figur 17: Schematische Darstellung des funktionellen Zusammenhangs zwischen β3-Endonexin-short, β3-Endonexin-long und weiteren intrazellulären und extrazellulären Komponenten im Fall eines Thrombozyt (Fig. 17A) sowie in einer Endothelzelle (Fig 17B).

Die folgenden Beispiele erläutern die Erfindung.

### Beispiel 1: Allgemeine Verfahren

### (A) Antikörper und Reagenzien

Der MAK Ab-15 ist gegen die extrazellulare Domäne von β3 gerichtet und wurde gemäß Standardverfahren mit Biotin konjugiert. Anti-IgG₁- und polyklonale Kaninchen-anti-GFP-Antikörper wurden als Fluorochrom-Konjugate (FITC oder "Texasrot" wie angegeben) von Dianova, Hamburg, bezogen. Phycoerythrin(PE)-konjugiertes Streptavidin wurde von Immunotech, Marseille, Frankreich, bezogen. Oligonukleotide wurden von MWG, Ebersberg, bezogen; Vitronectin stammte von Sigma, Deisenhofen/Taufkirchen.

### (B) cDNA-Klonierung und Mutagenese

Als Säuger-Expressionsvektor wurde P5C7, ein Derivat von pRK5, der eine modifizierte Polylinker-Region enthält, verwendet. Zytoplasmatische Ig-Fusionsproteine und die Transmembran-CD16/CD17-Chimären wurden wie kürzlich beschrieben hergestellt (Kolanus, W. et al., Cell 86 (1996), 233-242). Die Transmembran-Ig-Fusionsproteine umfassen die Leadersequenz von CD5, extrazelluläre CH2- und CH3-Domänen von humanem IgG₁, die CD7-Transmembran-Domäne und den intrazellulären Schwanz des β3-Integrin mit vollständiger Länge wie angegeben (Figur 1). Ein Ig-Konstrukt, dem die zytoplasmatische Domäne fehlt, diente als Kontrolle (Ig-Kontrolle). Die zytoplasmatischen Domänen von β1-A und β3-A wurden mittels PCR von Wildtyp-β1-A- und β3-cDNA-Sequenzen unter Verwendung von Primem mit den entsprechenden kodierenden Sequenzen und Restriktionsstellen am Anfang und Ende des open reading frame amplifiziert. Nach Spaltung mit Mlul und Notl wurden die entsprechenden PCR-Produkte in P5C7 kloniert. Die β3-B- und β3-C-Konstrukte wurden auf analoge Weise hergestellt. Die β3-A-Varianten V-A-NPKY, β3-A-Y759A, β3-A-1757P und die β1-A-Variante β1A-NITY wurden ebenfalls mittels PCR erzeugt.

Die zytoplasmatischen Domänen der β3-Integrine und deren Mutationen wurde über Amplifikation mittels PCR kloniert, wobei folgende Oligonukleotide verwendet wurden: β3-A: 5'-GGG GCG ACG CGT AAA CTC CTC ATC ACC ATC CAC-3' (vorwärts)/5'-GGG GCG GCG GCC GCT TTA AGT GCC CCG gTA CGT GAT ATT GGT GAA-3(rückwärts)'; β3-1757P: 5'-GGG GCG GCG GCG GCT TTA AGT GCG CCG GTA CGT ggg ATT GGT GAA GGT AG 3 (rückwärts)'; β3-Y759A: 5'-GGG GCG GCG GCG GCT TTA AGT GCC CCG GGC CGT GAT ATT GGT GAA GGT AG-3 (rückwärts)'; β3-NPKYEGK; 5'-GGG gCG GCG GCC GCT TTA TTT TCC CTC ATA CTT CGG ATT GGT GAA GGT AGA CGT GGC CTC-3' (rückwärts); β1-NITYRGT: 5'-Ggg GCG GCG GCC GCT TTA AGT GCG CCG GTA CGT GAT ATT GAC CAC AGT TGT TAC GGC ACT-3' (rückwärts).

Beide β3-Endonexin-Isoformen wurden von einer naturlichen Killerzellen-cDNA-Bank (Clontech, Heidelberg) über Amplifikation mittels PCR kloniert, wobei folgende Oligonukleotide verwendet wurden: 5'-GGG GCG ACG CGTATG ATG CCT GTT AAA AGA TCA CTG AAG TTG GAT GGT CTG-3' (vorwärts)/5'-GGG GCG GCG GCC GCT TCA CAG AGG TTG TGA CAT CTG AGG CTG AGG CTG ACC TTT GTG-3' (rückwärts) (β3-Endonexin-long) oder 5'-GGG GCG ACG CGT ATG ATG CCT GTT AAA AGA TCA CTG AAG TTG GAT GGT CTG-3' (vorwärts)/5'-GGG GCG GCG GCC GCT TCA CTG TAT ACT ACT TAA ATT TTG CAT TAT CTC CAT-3' (rückwärts) (β3-Endonexin-short). Die erhaltenen PCR-Fragmente wurden mit den entsprechenden 3-Termini einer eGFP-Klonierungs-Kassette (Clontech, Heidelberg) fusioniert. β3-Endonexin-Mutanten mit Deletionen der vermuteten nukleären Importsequenz K⁶²RKK wurden mittels einer Zwei-Schritt-PCR-Strategie erzeugt. Alle Konstrukte wurden mittels Restriktionsspaltung identifiziert, über CsCl-Zentrifugation gereinigt und mittels DNA-Sequenzierung vor der Transfektion überprüft.

Ebenso wurden β3-Endonexine als Konstrukte ohne eGFP in den gleichen Expressionsvektor kloniert.

### (C) Zellinien und transiente Infektionen

CHO-Zellen stammten von der American Type Culture Collection (ATCC, Rockville, MD) und wurden in nach Dulbecco modifiziertem Eagle-Medium (DMEM, Sigma) gehalten, das mit 10% fötalem Kälberserum (FCS), 1% L-Glutamin, 1% Penicillin und Streptomycin und 1 % nichtessentiellen Aminosäuren supplementiert worden war. Stabile α_{IIb}β3 und α_{IIb}β3 (Y759A) exprimierende CHO-Zellinien wurden bereits früher charakterisiert (Ylänne, J., et al., J. Biol. Chem. 270 (1995), 9550-9557). Die Zellinien wurden in DMEM (Sigma) kultiviert, das mit 0,75 mg/ml Geneticin (G418)-Disulfat supplementiert worden war.

Human umbilikale Endotheizellen (HUVEC) wurden bei Clonetics, Kalifornien, USA, gekauft. Sie wurden in "Endothelial Basal Medium" (vom selben Hersteller) kultiviert und maximal 5x passagiert.

cDNA-Konstrukte wurden in CHO-Zellen mittels Liposomen-vermittelter Transfektion (Superfect™, Quiagen, Hilden) exprimiert. 24 Stunden vor Transfektion wurden die Zellen auf Kulturplatten mit sechs Vertiefungen plattiert. Insgesamt 2 µg von jedem Konstrukt und 10 µl Superfect™ (Quiagen) wurden bei Raumtemperatur 10 Min. in 110 µl nicht-supplementiertem DMEM bzw. M-199 (Gibco Life, Karlsruhe) inkubiert. Danach wurden 600 µl supplementiertes Medium dazugegeben und die DNA-Superfect™-Komplexe auf die Zellen überlagert. Die Zellen wurden 2 Stunden bei 37°C inkubiert, mit PBS gewaschen und danach bei 37°C mit komplettem Medium inkubiert. Nach 24 Stunden wurde das Medium gewechselt und die Analyse der Zellen erfolgte bei 48 Stunden.

### (D) Durchflusszytometrie und konfokale Laser- Immunfluoreszenz-Mikroskopie

Transiente Transfektanten wurden in Tyrode-Puffer geerntet, der 0,1 mg/ml mit L-1-Tosylamido-2-phenylethylchloromethyl-Keton behandeltes Trypsin (Sigma) und 3,5 mM EDTA enthielt. Nach 5 Min. Inkubation bei Raumtemperatur wurden die Zellen mit Tyrode-Puffer verdünnt, der 0,1 % Sojabohnen-Trypsin-Inhibitor (Sigma) und 10% Rinderserumalbumin (BSA) enthielt, und durch die Zugabe eines gleichen Volumens an 2% Formaldehyd in PBS für 30 Min. bei Raumtemperatur fixiert. Danach wurden die Zellen durch Zentrifugation bei 1200 UpM (5 Min.) gewonnen und einmal in Tyrode-Puffer gewaschen. Für die Oberflächen-IgG-Färbung wurden die fixierten Zellen in 100 µl Tyrode-Puffer resuspendiert, der einen Überschuss an Phycoerythrin(PE)-konjugierten Maus-anti-tgG₁-MAK (50 µg/ml) enthielt. Nach einstündiger Inkubation wurden die Zellen zweimal mit 2% Glycin in PBS gewaschen und für die intrazelluläre Ig-Färbung erneut in Tyrode-Puffer resuspendiert, der Fluorescein(FITC)-konjugierte Maus-anti-IgG,-MAK (50 µg/ml) und 0,2% Triton X-100™ zur Permeabilisierung der fixierten Zellen enthielt. Nach einer weiteren einstündigen Inkubation wurden die Zellen wieder zweimal gewaschen, in 0,5 ml Tyrode-Puffer resuspendiert und mittels Durchflusszytometrie auf einem "FACScan" (Becton Dickinson, Heidelberg) analysiert. 10.000 grünfluoreszente Transfektanten (positiv für intrazellulare FITC-anti-IgG-Färbung) wurden hinsichtlich roter Immunfluoreszenz (PE) (Oberflächen-PE-anti-IgG-Färbung) analysiert. In Experimenten mit GFP-markierten Proteinen wurden die fixierten Zellen mit PE-anti-IgG ohne Permeabilisierung oberflächengefärbt. Für die konfokale Laserimmunfluoreszenz-Mikroskopie wurden transfizierte Zellen auf mit Vitronectin (5 µg/ml) beschichteten Deckgläschen bei 4°C über Nacht inkubiert und 1 Stunde mit 5% BSA in PBS bei Raumtemperatur geblockt. Danach wurden Zellmonolayer für die Durchflusszytometrie-Analyse wie vorstehend beschrieben fixiert und gefärbt. Die Immunfluoreszenz-Analyse wurde mittels eines konfokalen Laser-Mikroskops (Leica, Bensheim) durchgeführt, das mit entsprechender Software ausgestattet war.

### (E) Zellyse und Immunpräzipitation

Für die Immunpräzipitation wurden subkonfluente Monolayer von CHO-Zellen mit Ig- oder GFP-Fusions-Konstrukten transient transfiziert. Zellen wurden durch Zugabe von Lyse-Puffer lysiert, der 100 mM Tris, pH Wert 8,0, 150 mM NaCl, 2 mM EDTA und 1 % Triton X-100T™ enthielt. Nach 20minütiger Inkubation bei 4°C wurden zur Entfernung von unlöslichem Material die Lysate bei 20.000 x g zentrifugiert. Danach wurden Ig-Fusionsproteine mittels Protein A-6MB-Sepharose-Perlen (Amersham Pharmacia Biotech., Freiburg) direkt gewonnen. Immunpräzipitate wurden vor der Dissoziation in SDS-Auftragspuffer dreimal mit Lyse-Puffer gewaschen. Die Proteine wurden auf SDS-Polyacrylamidgelen aufgetrennt und auf Nitrozellulose-Membranen überführt. Der immunologische Nachweis erfolgte mittels Peroxidase-konjugierten sekundären Antikörpern (Dianova) und Chemilumineszenz (Amersham Pharmacia Biotech).

### (F) Endozytose

Die Endozytose von β3-Integrinen wurde mittels eines nach Bretscher (1992) modifizierten Verfahrens gemessen (Bretscher, M.S., EMBO J. 11 (1992), 405-410). Ähnliche Spiegel an α_{IIb}β3 und α_{IIb}β3 (Y759A) exprimierende stabile Zellen wurden über Nacht auf Platten mit einem Durchmesser von 35 mm kultiviert, zweimal mit Dulbecco-modifiziertern PBS (Life Technologies, Karlsruhe) gewaschen, mit Biotin-markiertem anti-β3-MAK (5 µg/ml) 15 Min. auf Eis vorinkubiert und weitere 0, 2, 5, 15, 30 oder 60 Min. bei 37°C in Dulbecco-modifizierter PBS inkubiert. Danach wurden die Zellen zweimal gewaschen, worauf sich drei aufeinanderfolgende 5-minütige Inkubationen mit einer reduzierenden Lösung anschlossen, die 50 mM 2-Mercaptoethansulfonsäure (Sigma), 10 mM NaCl, 1 mM EDTA, 50 mM Tris und 0,2% Rinderserumalbumin bei einem pH-Wert von 8,6 enthielt. Die Zellen wurden abgelöst, und ein Aliquot davon wurde mit 2% Formaldehyd 30 Min. auf Eis fixiert, zweimal mit 2% Glycin gewaschen und mit PE-Strepatavidin in Gegenwart von 0,2% Triton-X 100_{TM} weitere 30 Min. inkubiert. Danach wurden die Zellen mittels Durchflusszytometrie analysiert, und die mittlere Intensität von PE-Streptavidin von 10.000 Ereignissen wurde als Maß für die β3-Integrin-Endozytose verwendet. Ein weiteres Aliquot von CHO-Zellen wurde mit Biotin-konjugiertem anti-β3-MAK für die angegebene Zeit inkubiert, nach Reduktion mit 2-Mercaptoethansulfonsäure mit 200 µl 200 mM n-Octly-β-D-Glucopyranosid (Sigma), 1 mM Phenylmethylsulfonylfluorid (Sigma) in Dulbecco-modifizierter PBS bei 4°C lysiert und 5 Min. bei 12.000 x g zentrifugiert. Danach wurde das gleiche Volumen 100 mM Tris, 150 mM NaCl, 1 mM CaCl₂, 1% Triton X-100™, 0,1% SDS und 0,1% Nonidet P-40, pH Wert 7,4, zugegeben. Schließlich wurden 20 µg Protein pro Spur auf 8% Acrylamid-Flachgele gegeben, und nach Auftrennung unter nicht-reduzierenden Bedingungen wurden die Proteine unter Verwendung von 25 mM Tris, 192 mM Glycin, 20% Methanol, pH-Wert 8,3 (Ylänne, J., et al., supra) auf eine Immobilon™-Membran (Millipore, Eschborn) überführt. Biotin-markierte anti-β3-MAK wurden mittels 2 µg/ml Peroxidase-gekoppeltem Streptavidin (Dianova) und einem Chemilumineszenz-Immunblot-Kit (Roche Diagnostics, Mannheim) sichtbar gemacht.

### Gel Shift Assays zur Messung der NF-kB-Aktivität:

Kernextrakte wurden aus HUVEC präpariert und wie beschrieben analysiert. Das prototypische Immunglobulin-Kappa-Ketten-Oligonukleotid wurde als Sonde benutzt und mit Klenow-Fragment DNA-Polymerase I (Roche, Mannheim) markiert. Nukleäre Proteine wurden mit ³²P-dCTP 30 Minuten bei 25°C in 20 µl Bindungspuffer inkubiert. Die Proben wurden in 0.25% TBE (TRIS, Borat, EDTA, pH 8.0) auf nichtdenaturierenden 4%-igen Polyacrylamid-Gelen aufgetrennt. Die Gele wurden dann getrocknet und autoradiographiert.

### (G) Transfektionen

Reporterplasmide wurden mit dem SuperFect™ Transfektionsreagenz (Qiagen, Hilden, Deutschland) mit 3 x 10⁵ CHO-Zellen transfiziert und über Nacht in Platten mit 6 Vertiefungen geimpft. Für die Chloramphenicol-Acetyltransferase (CAT)-Assays wurden alle transienten Transfektionen in Gegenwart von 1 µg u-PAR-CAT-Reporterkonstrukten und 1 µg Luziferase-Expressionsvektor (siehe nächster Absatz) durchgeführt und, wenn angegeben, zusätzlich mit 3 µg Expressionsplasmid, das für β3-Integrin kodiert, oder äquimolaren Mengen des Kontrollvektors. Nach drei Stunden wurden die Zellen zweimal mit PBS gespült, dann wurde zu 10% FCS enthaltendem Medium gewechselt und weitere 45 Stunden gezüchtet. Die Zellen wurden geerntet und danach durch wiederholte Gefrier/Auftau-Zyklen in 0,25 M Tris-HCl, pH-Wert 7,8, lysiert. Die Transfektionseffizienz wurde mittels Luziferase-Aktivitätsassays bestimmt. Nach Normalisierung für die Transfektionseffizienz wurde die CAT-Aktivität durch Inkubation der Zellysate mit 4 µM [¹⁴C]-Chloramphenicol und 1 mg/ml Acetyl-Coenzym A bei 37°C gemessen. Das Gemisch wurde durch Extraktion mit Ethylacetat abgetrennt und die acetylierten Produkte wurden auf Dunnschichtchromatographie-Platten unter Verwendung von Chloroform/Methanol als mobile Phase aufgetrennt. Die Reaktionsprodukte wurden mittels Autoradiographie sichtbar gemacht und die Radioaktivität wurde mittels eines "445 SI Phosphor Imager" (Molecular Dynamics) quantifiziert.

Für Luziferase-Assays wurden die transienten Transfektionen in Gegenwart von 1 µg u-PAR-Luziferase-Reporterkonstrukt und 10 ng eines Renilla-Luziferase-Plasmids (pRL-SV40; Promega, *Mannheim)* als interne Kontrolle zusammen mit 3 µg β3-Integrin kodierendem Expressionsplasmid oder dem Kontrollvektor durchgeführt. Nach der Transfektion wurden die Zellen drei Stunden in serumfreiem Medium gezüchtet und danach weitere 45 Stunden zusätzlich mit 10% FCS. Leuchtkäfer-Luziferase-Aktivität wurde für Renilla-Luziferase standardisiert, die als interne Kontrolle venwendet wurde.

### Beispiel 2:

### Isoform-abhängige differentielle Zelloberflächenexpression von β3-Integrin-Chimären

β3-Integrine sind an wichtigen biologischen Prozessen beteiligt, wobei davon ausgegangen wird, dass die zytoplasmatischen Domänen der β-Untereinheiten beispielsweise bei der Regulation der extrazellulären Bindung von Liganden ("inside-out-signaling"), Signaltransduktion und Internalisierung/Recycling der Rezeptoren eine Rolle spielen. Zum Verständnis der funktionellen Signifikanz der drei bekannten zytoplasmatischen β3-Domänen-Isoformen wurden daher einzelkettige chimäre Rezeptoren erzeugt, die CH2- und CH3-Anteile von humanem IgG1 an der Zelloberfläche tragen, die Transmembran-Domäne von CD7 und die zytoplasmatischen Domänen β3-A, -B bzw. C mit vollständiger Länge (Figur 1). Ein analoges Konstrukt, das die zytoplasmatische Domäne von β1-A trägt, oder ein schwanzloses Ig-Konstrukt wurden als weitere Kontrollen verwendet. Alle Konstrukt basierten auf kürzlich beschriebenen chimären Rezeptoren (Kolanus, W., et al., Cell 74 (1993) 171-183). Bei Expression dieser Chimären in verschiedenen Zellsystemen stellte sich heraus, dass die β3-A-Chimären im Vergleich zu anderen β3-Isoform-Fusionsproteinen oder Kontroll-Chimären eine drastisch erniedrigte Zelloberflächen-Expression zeigten. CHO-Zellen (Figur 2A) wurden mit Ig-Chimären transient transfiziert und die Zellen zum Nachweis sowohl der Zelloberflächenfraktion als auch der intrazellulären Expression der Fusionsproteine sequentiell mit Fluorescein- oder Texas-Rot-gekoppelten anti-Ig-Antikörpern gefärbt. Wie in Figur 2A gezeigt, zeigten Ig-β3-B, Ig-β3-C und Ig-β1-A ein diffuses intrazelluläres und "steady state"-Plasmamemembran-Oberflächenfärbungsmuster, das dem Färbungsmuster der schwanzlosen Ig-Chimären ähnelte. Im Gegensatz dazu war das Ig-β3-A primär intrazellulär lokalisiert (Figur 2A). Zur quantitativeren Abschätzung der Oberflächenexpression der Fusionsproteine wurden CHO-Zellen mit den entsprechenden Konstrukten transient transfiziert, fixiert und hinsichtlich intrazellulärer und Oberflächenexpression doppelgefärbt mittels Durchflusszytometrie untersucht. Wie in Figur 2B gezeigt war die Oberflächenexpression von Ig-β3-A im Vergleich zu den Mutanten mit alternativen Spleißstellen (Ig-β3-B, Ig-β3-C), den Ig-β1-Chimären oder dem schwanzloson Ig-Konstrukt signifikant verringert. Die Unterschiede in der Oberflächenexpression der getesteten Chimären beruhte nicht auf Unterschieden der Gesamtproteinkonzentration, da die mittlere Intensität der intrazellulären Immunfluoreszenz für alle transfizierten Konstrukte gleich war, und die Immunblot-Analyse bestätigte, dass die "steady state"-Expressionsspiegel aller getesteten Chimären sehr ähnlich waren (Figur 2C). Diese Ergebnisse implizieren daher, dass die zytoplasmatische β3-A-Domäne an der Regulation der Zelloberflächenexpression dieses speziellen β3-Integrins beteiligt ist.

### Beispiel 3:

### Das NITY-Motiv bei β3-A 756-759 ist für die Oberflächenexpression der Ig-β3-Chimären kritisch

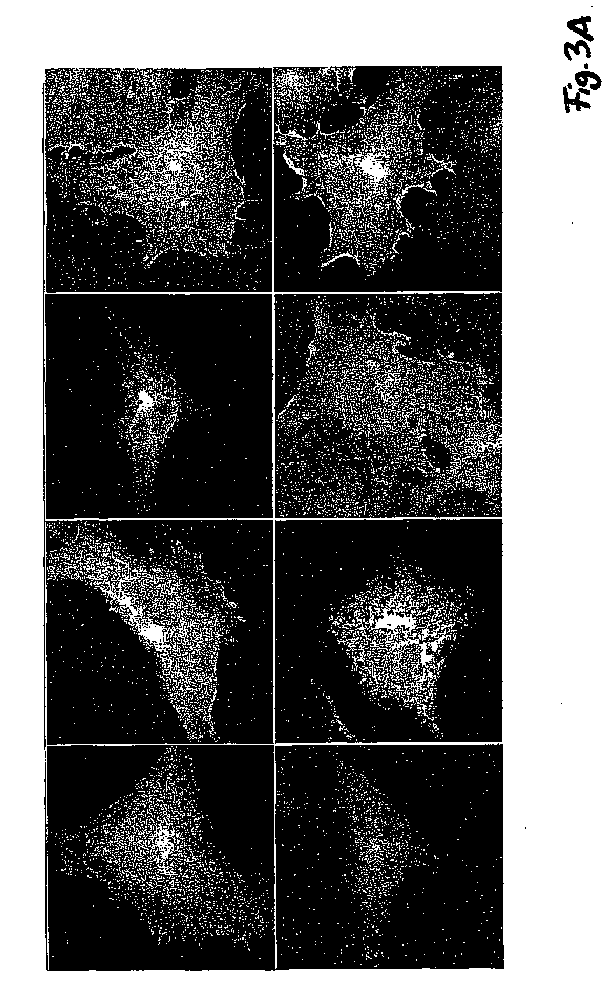

Die zytoplasmatischen Domänen der β3-Integrine interagieren mit einer Reihe von Proteinen des Zytoskeletts und Signalproteinen. Konservierte Bereiche, wie z.B. NPXY und NXXY, die bei den meisten zytoplasmatischen β-Domänen vorhanden sind, vermitteln die Lokalisation der β3-Integrine innerhalb der Plasmamembran während der Ausbreitung der Zellen und der Bildung herzförmiger Adhäsion. Es stellte sich daher die Frage, ob Mutationen oder Deletionen des C-terminalen NlTY-Motivs, das ausschließlich in der zytoplasmatischen β3-A-Sequenz exprimiert wind (Figur 1), die Oberflächenexpression der entsprechenden Chimären beeinflussen. Tatsächlich zeigte sich, dass eine Substitution des Carboxy-Terminus von β1 gegen die C-terminalen sieben Reste der zytoplasmatischen β3-Domäne (756-762) (NITYRGT, Figur 1) oder, alternativ, die Substitution des NITYRGT-Motivs in β3-A gegen das NPKYEGK-Motiv (772-778) von β1-A (Figur 1) zu einer wesentlich erhöhten Oberflächenexpresssion der Ig-β3-A-NPKY-Mutante und einer gleichzeitigen Abnahme des Oberflächennachweises der Ig-β1-A-NITY-Chimäre führte (Figuren 3A und 3B). Außerdem erhöhte die Einführung von Pro⁷⁷³ von β1-A in die zytoplasmatische β3-A-Domäne (β3-A-I757P) die Oberflächenexpression der Chimären wesentlich (Figuren 3A und 3B). Ähnlich Ergebnisse mit erhöhter Zelloberflächenexpression wurden mit einer Alanin-Substitution bei Tyr⁷⁵⁹ (β3-A-Y759A) in β3-A erhalten (Figuren 3A und 3b). Diese Daten zeigen, dass das lineare Sequenzmotiv von β3-A N⁷⁵⁶ITY für eine verringerte Zelloberflächenexpression des β3-A-β3-Integrin-Fusionsproteins von kritischer Bedeutung ist. Das N⁷⁵⁶ITY-Motiv ist auch für die "inside-out"-Signalübertragung in Blutplättchen und CHO-Zellen erforderlich und vermittelt die Interaktion mit dem zytoplasmatischen Protein β3-Endonexin.

### Beispiel 4:

### β3-Endonexin reguliert die Oberflächenexpression von Ig-β3-Fusionsproteinen

β3-Endonexin bindet an die zytoplasmatische Domäne der β3-A-Untereinheit. Diese Interaktion ist spezifisch, da sie nicht bei den zytoplasmatischen Domänen anderer β3-Integrine beobachtet wurde. Es konnte außerdem gezeigt werden, dass die Bindung von β3-Endonexin an β3-A von dem N⁷⁵⁶ITY-Motiv, das nur in β3-A vorhanden ist, abhängt (Figur 1). Es wurden zwei Isoformen von β3-Endonexin beschrieben, die sich hinsichtlich der Größe unterscheiden, und, was von größerer Bedeutung ist, hinsichtlich ihrer Kapazität zur Bindung an die zytoplasmatische Domäne von β3-A (Figur 4A). Die "short" β3-Endonexin-Spleißvariante (β3-Endonexin-S; En-S) besteht aus 111 Aminosäuren und bindet an β3-A, während die längere Form von β3-Endonexin (170 Aminosäuren, β3-Endonexin-L; En-L) nicht mit dem β3-A-Schwanz interagiert (siehe das Schema in Figur 4A). Da die N-Termini beider Polypeptidvarianten identisch sind, wurden diese beiden Isoformen verwendet, um zu untersuchen, ob β3-Endonexin an der Regulation der Zelloberflächenexpression von β3-A beteiligt ist. Zur Untersuchung der Auswirkung beider Isoformen von β3-Endonexinen auf die Zelloberflächenexpression von β3-A wurden cDNAs für beide Isoformen aus einer naturlichen Killerzellinien-cDNA-Bank isoliert und für den üblichen subzellulären Nachweis an den C-Terminus des "green fluorescent protein" (GFP) fusioniert. Da GFP/β3-Endonexin sowohl im Zytoplasma auch im Nucleus exprimiert wird, wurde beide Mutanten der beiden β3-Endonexin-Isoformen, die konstitutiv im Zytoplasma lokalisiert sind, konstruiert, d.h., bei denen das vermutete Signal für die nukleären Lokalisation an Position K⁶²RKK deletiert wurde. Wie in Figur 4C gezeigt, zeigten Mutanten hinsichtlich des nukleären Imports beider β3-Endonexin-Isoformen (En-SΔK⁶²RKK und En-LΔK⁶²RKK) eine im Vergleich zu den Wildtyp-β3-Endonexin-Chimären eine wesentlich stärkere zytoplasmatische GFP-Fluoreszenz. Bei Cotransfektion der Wildtyp- und mutierten β3-Endonexin-Isoformen (Figur 4C) mit Ig-β3-Integrin-Chimären in CHO-Zellen oder HUVEC zeigte sich, dass die Oberflächenexpression von Ig-β3-A in mit der langen Form von β3-Endonexin cotransformierten Zellen im Vergleich zu Zellen, die mit der kurzen Form von β3-Endonexin oder mit Kontrollkonstrukten cotransformiert worden waren, deutlich erhöht war (Figuren 4C und 4D). Die Oberflächenexpression von β3-A war dann etwas stärker erhöht, wenn die Zellen mit der nukleären Importmutante der langen Form von β3-Endonexin (En-LΔK⁶²RKK) cotransfiziert worden waren. Die Kurzform-Mutante (En-SΔK⁶²RKK) zeigte keinen signifikanten Effekt auf die Zelloberflächenexpression von Ig-β3-A (Figuren 4C und 4D). Bei der Cotransfektion von GFP/β3-Endonexin und den Ig-β1-A-NITY-Chimären wurde ähnlich beobachtet, dass En-L die Oberflächenexpression dieses Fusionsproteins signifikant erhöht (Figuren 4C und 4D), während kein substantieller Effekt beider β3-Endonexin-Isoformen auf die Oberflächenexpression der anderen Ig-β3-Integrin-Chimären beobachtet wurde (Figuren 4C und 4D). Dieser Befand legt eindeutig nahe, dass das β3-Endonexin-System die Zelloberflächenexpression von β3-A spezifisch reguliert. Die Tatsache, dass beide GFP/β3-Endonexine primär im Nucleus lokalisiert rind, scheint in diesem Zusammenhang keine wesentliche Rolle zu spielen: Obwohl die konstitutive zytoplasmatische Lokalisation von GFP-β3-Endonexin-L die Plasmamembran-Expression von β3-A etwas besser erhöht als die Wildtypversion, zeigen beide Konstrukte qualitativ ähnliche Auswirkungen.

### Beispiel 5

### β3-A-Integrin-Fusionsproteine werden über einen β3-Endonexin abhängigen Mechanismus internalisiert

Es ist bekannt, dass β3-Integrine über Endozytose internalisiert werden, wobei die zytoplasmatischen Domänen bei diesem Prozess eine wichtige Rolle spielen. Zur Abschätzung der Rolle der zytoplasmatischen Domänen von β3-Integrin bei dem endozytotischen Mechanismus wurden CHO-Zellen transient mit Ig-β3-Integrin-Chimären transfiziert. Nach 24 Stunden wurde ein Fluorochrom-markierter anti-Ig-MAK 15 Min. zu den lebenden Zellen gegeben. Danach wurde der Antikörper entfernt und die Zellen wurden mit 2% Formaldehyd zu den angegebenen Zeiten fixiert. Es zeigte sich, dass der anti-Ig-Antikörper in β3 A-transfizierten Zellen rasch internalisiert wurde und innerhalb von Minuten in einem vesikulären Kompartiment unterhalb der Plasmamembran nachgewiesen werden konnte (Figur 5A). Eine längere Inkubationszeit führte zur Zentralisierung der anti-Ig-FITC-Immunfluoreszenz. Ähnliche Ergebnisse zeigten sich mit Ig-β1-Konstrukten, die das N⁷⁵⁶ITY-Motiv von β3-A enthielten. Im Gegensatz dazu wurde eine vorherrschende oder ausschließliche Oberflächenfärbung mit praktisch keiner oder lediglich minimaler zellulären Aufnahme von anti-Ig-MAK in Zellen beobachtet, die mit den anderen Isoformen β3-B oder β3-C, den β1-A- oder schwanzlosen Ig-Chimären transient transfiziert worden waren (Figur 5A). Diese Ergebnisse zeigen, dass - obwohl die "steady state"-Oberflächenexpression von β3-A stark verringert ist (Figuren 2A und 2B) - ein Anteil des zellulären Pools der Ig-β3-A-Fusionsproteine offensichtlich zwischen der Plasmamembran und einem intrazellulären vesikulären Kompartiment zykliert. Im Gegensatz dazu zeigten Zellen, die mit Punktmutanten von β3-A, β3-A-Y759A und β3-A-1757P oder mit anderen β3-Isoformen transfiziert worden waren, die eine wesentliche "steady state"-Oberflächenexpression zeigten (Figuren 2 und 3), eine wesentlich geringere Internalisierung des anti-Ig-Antikörpers (Figur 5A). Daher kann geschlossen werden, dass das N⁷⁵⁶ITY-Motiv an der Internalisierung oder Endozytose des β3-A-Integrins beteiligt ist.

Danach wurde untersucht, ob β3-Endonexin die Internalisierung von β3-A moduliert. Dazu wurden CHO-Zellen mit den β3-Endonexin-Isoform-GFP-Fusionsproteinen oder Mutanten davon und einer Ig-β3-Chimären cotransfiziert. Die Aufnahme eines FITC-konjugierten anti-Ig-MAK wurde mittels Immunfluoreszenz-Mikroskopie zu den angegebenen Zeitintervallen überwacht. Es zeigte sich, dass in Zellen, die mit den Chimären der langen Isoform und Ig-β3-A cotransfiziert worden waren, die Aufnahme des FITC-anti-Ig-MAK im Vergleich zu mit En-S-Konstrukten cotransfizierten Zellen wesentlich weniger ausgeprägt war (Figur 5B). Diese Ergebnisse zeigen, dass die Internalisierung von anti-Ig-MAK-ligierten β3-A-Integrinen durch das zytoplasmatische β3 A-bindende Protein β3-Endonexin reguliert wind.

### Beispiel 6

### Anti-β3-MAK-induzierte Endozytose nativer β3-Integrine wird über einen β3-Endonexin-abhängigen Mechanismus reguliert.

Um zu untersuchen, ob β3-Endonexin an der Internalisierung des nativen β3-Integrins beteiligt ist, wurden Wildtyp-α_{IIb}β3 und α_{IIb}β3(Y759A) exprimierende Zellinien mit einem Biotin-markierten anti-β3-MAK entweder auf Eis oder bei 37°C zu den angegebenen Zeitabständen inkubiert und einem Membran-undurchlässigen Reduktionsmittel ausgesetzt. Diese Verbindung setzte den Streptavidin-FITC-vermittelten Nachweis des Antikörpers außer Kraft und stellte daher ein Mittel zur Messung der Internalisierung des Rezeptors bereit.

Figur 6A dokumentiert, dass Wildtyp- und Mutanten-β3-Integrine mit ähnlicher Dichte auf der Oberfläche von CHO-Zellen exprimiert wurden. Sich daran anschließende Immunblot-Experimente zeigten, dass ungefähr 40% des Biotin-markierten anti-β3-Antikörpers, der bei 4°C an die Zelloberfläche gebunden war, vor der Reduktion geschützt waren, wenn Wildtyp-α_{IIb}β3 exprimierende Zellen verwendet wurden (Figuren 6B und 6C). In α_{IIb}β3 (Y759A) exprimierenden Zellen waren jedoch lediglich 5-6% des bei Kälte gebundenen Materials vor Reduktion geschützt (Figuren 6B und 6C), was anzeigt, dass im Wildtyp wesentliche Endozytose eintrat, jedoch nicht in Mutanten-β3 exprimierenden Zellen. Somit werden - wie vorstehend für die β3-Integrin-Chimären beschrieben - die zytoplasmatischen Domänen von nativem β3-Integrin und die NITY⁷⁵⁹⁻Sequenz für eine signifikante Endozytose von α_{IIb} β3 in CHO-Zellen benötigt.

Analoge Experimente wurden dann unter Verwendung von Immunfluoreszenz-Verfahren durchgeführt. Mikroskopische Analysen zeigten, dass wesentliche Mengen eines Oberflächen-gebundenen, Biotin-markierten MAK zu intrazellulären Stellen in Wildtyp α_{IIb} β3 exprimierenden Zellen überführt wurden (Figur 7B, linker Teil). Im Gegensatz dazu war die Internalisierung des Biotin-markierten anti-β3-MAK in α_{IIb}β3(Y759A) exprimierenden Zellen signifikant verringert (Figur 7B, rechter Teil). In der Mutante (Figur 7B, rechter Teil) war der zum Nachweis verwendete Antikörper niemals in großen vesikulären Strukturen nachweisbar so wie dies in den Wildtyp-β3 exprimierenden Zellen beobachtet werden konnte. Jeder Oberflächen-gebundene Antikörper, der geschützt wurde, blieb überhaupt mit dem Zellkortex assoziiert.

Um eine quantitativere Abschätzung der mittels Immunfluoreszenz gemessenen β3-Integrin-abhängigen Internalisierung zu erhalten, wurden die Zellen mittels Durchflusszytometrie untersucht. Wie in Figur 7A dargestellt war die Aufnahme der PE-Streptavidin-Fluoreszenz in α_{IIb}β3(Y759A) (Kreise) exprimierenden Zellen im Vergleich zu Wildtyp-α_{IIb}β3 exprimierenden Zeilen (Quadrate) signifikant verringert.

### Beispiel 7:

### Die transiente Überexpression von GFP-Endonexin-L hemmt die Endozytose von α_{IIb}β3 in CHO-Zellen

Um abzuschätzen, ob die Überexpression von β3-Endonexin auch die Endozytose des nativen β3-Integrins moduliert, wurden α_{IIb}β3 oder α_{IIb}β3(Y759A) exprimierende Zellen mit verschiedenen GFP/β3-Endonexin-Fusionskonstrukten transient transfiziert und mit Biotin-markiertem anti-β3-AK 15 Min. bei 37°C inkubiert. Nach Reduktion mit 2-Mercaptoethansulfonsäure wurden die Zellen mit PE-Streptavidin gefärbt und mittels Durchflusszytometrie untersucht. In Übereinstimmung mit den Experimenten, bei denen β3-A-Chimären verwendet wurden, zeigte sich, dass die Expression der langen Form von β3-Endonexin oder der Deletionsmutante En-LΔK⁶²RKK zu einer Dosisabhängigen signifikanten Hemmung der PE-Streptavidin-Fluoreszenz-Aufnahme führte, während En-S keine Auswirkung zeigte. Diese Ergebnisse zeigen, dass Endozytose des nativen β3-Integrin durch das zytoplasmatische β3-Endonexin reguliert wird (Fig. 8).

### Beispiel 8

### β3-Integrin-Internalisierung in Speichervesikel (α-Granula) von Thrombozyten aus Patienten mit koronarer Herzerkrankung

Um zu untersuchen, inwiefern β3-Integrine an menschlichen Thrombozyten (GPIIb-IIIa) in α-Granula internalisiert werden, wurde thrombozytenreiches Plasma aus mit saurer Citrat-Dextrose (ACD; nach der Formel A des National Institute of Health, Bethesda, Maryland, USA) antikoaguliertem Blut von Patienten mit koronarer Herzerkrankung durch Zentrifugation bei 230xg über 16 Minuten abgetrennt. Dann wurden die Thrombozyten gewaschen und in einem Medium mit 90 mM NaCl, 5 mM KCI, 2 mM CaCl₂, 1 mM MgCl₂, 5 mM Glucose, 30 mM Phosphatpuffer pH 6.5 resuspendiert. Die Thrombozyten wurden mit 20 µg/ml 7E3 monoklonalem Antikörper (inhibiert die Fibrinogen-Bindungsstelle des GP IIb/IIIa und wurde von Dr. Coller, New York, USA zur Verfügung gestellt) 5 Minuten bei 20°C inkubiert. Als Negativkontrolle wurde mit 20 µg/ml unspezifischem Maus-IgG inkubiert. Nach erneuter Zentrifugation (5 Min., 180xg) und Resuspension in 120 mM NaCl, 5 mM KCl, 1 mM CaCl₂, 0.1 mM Mg Cl₂, 5 mM Glucose, 1 mM Phosphatpuffer und 30 mM TES-Puffer bei pH 7.4 wurden 10 µg/ml 5nm-Gold-markierte anti-Maus-IgG Fab-Fragmente (Biotrend, Köln) zugefügt. Die Suspension wurde 10, 20, 60 und 90 Minuten inkubiert. Dann wurden die immunmarkierten Proben mit 0.2% Glutaraldehyd fixiert und für die Elektronenmikroskopie wie beschrieben vorbereitet.

Der Zeitverlauf, der in den Figuren 9A-C dargestellt ist, zeigt, dass thrombozytäre α-Granula wesentlich von der Zell-Oberfläche internalisierten Glykoproteinen IIb/IIIa, den β3-Integrinrezeptoren des Thrombozyten, ummantelt werden. Daraus wird deutlich, dass eine Inhibition der β3-Integrin-Inhibierung wesentliche Auswirkung auf die Zusammensetzung und die Funktion der Speichervesikel hat.

### Beispiel 9

### β3-Integrin-Internalisierung in α-Granula bewirkt verminderte α-Degranulation

Um anhand der P-Selektin-Oberflächenexpression auf humanen Thrombozyten deren degranulatorische Aktivität und damit die Freisetzung vasoaktiver und atherogener Mediatoren aus α-Granula abschätzen zu können, wurden Thrombozyten-reiche Plasmen aus zitiertem Vollblut durch Zentrifugation angereichert (200 x g über 20 Minuten). Die endgültig Thrombozytenzahl wurde mittels autologem Plasma auf 2 x 10⁸/ml eingestellt. Nach Festlegung der Nullwerte wurden 5 µl TRAP (25 µM) zugegeben; die P-Selektin-Oberflächenexpression wurde mittels Durchflusszytometrie-Methode (beschrieben in: Gawaz M, et al., Thromb Haemost 80 (1998), 994-1001) bestimmt. Verwendet wurde dazu ein monoklonaler anti-CD62P-Antikörper (Klan CLBThromb/6) der Firma Immunotech, Marseille, Frankreich, der spezifisch P-Selektin auf der Oberfläche degranulierter Thrombozyten erkennt.

Es zeigte sich (Figur 10), dass die P-Selektin-Oberflächenexpression/Sekretion aus Thrombozyten (als Beispiel für die Sekretion verschiedener vasoaktiver Mediatoren, wie Serotonin, Wachstumsfaktoren oder Cytokine) abhängig ist von der Internalisierung ihrer oberflächenständigen β3-Integrine, der Glykoprotein IIb/IIIa-Rezeptoren, die zeitgleich auftritt (Figur 9).

Eine verstärkte β3-Integrin-Internalisierung vermindert zeitabhängig die Sekretion vasoaktiver Substanzen, weil die Mediatoren-Wiederaufnahme-Kapazität der α-Granula sinkt. Damit kann eine direkte Förderung dieses Internatisierungsvorgangs, wie durch β3-Endonexin-short-Überexpression/-Aktivierung oder Endonexin-long-Hemmung möglich, diese Sekretion deutlich vermindern.

### Beispiel 10

### β3-Endonexin-short reguliert den uPAR-Promotor herunter

3 x 10⁵ CHO-Zellen wurden über Nacht in 6-well-Schalen ausgesät, dann transient mittels SuperFect-Transfektionsreagenzes (Qiagen, Hilden) transfiziert. Für die Chloramphenicol-Acetyltransferase-Versuche wurde dabei 1 µg eines CAT-Reporterkonstruktes verwendet, das unter der Kontrolle des Urokinase-type Plasminogen Activator Receptor-(uPAR)-Promotors stand. Dazu wurde ein 449 bp-Fragment des uPAR-Gens (Sequenzposition -398 bis +51) in die Xbal-Schnittstelle des pCAT-Basic-Vektors (Promega, Mannheim) kloniert. Zusätzlich wurden je 1 µg Luciferase-Expressionsvektor und verschiedene Mengen von β3-Endonexin-short-Plasmid (200 ng bis 5 µg) oder dem leeren Expressionsvektor (pEGFP) in äquimolarer Menge kotransfiziert. Nach 3 Stunden wurden die Zellen zweimal mit PBS gewaschen, in 10%-Fötalkälberserum (Gibco, Karlsruhe)-haltiges Medium überführt und für weitere 45 h kultiviert. Die Zellen wurden dann geerntet und mittels wiederholter Gefrier-Tau-Zyklen in 0.25 M Tris-HCl, pH 7.8, lysiert. Zellextrakte, die hinsichtlich der Luziferase-Aktivität normalisiert worden waren, wurden mit 4 µM ¹⁴C-Chloramphenicol und 1 mg/ml Acetyl-Coenzym inkubiert, mit Ethylacetat extrahiert und einer Dünnschichtchromatographie unter Verwendung von Chloroform/Methanol als mobiler Phase unterzogen. Die Umwandlung von ¹⁴C-Chloramphenicol zu acetylierten Derivaten wurde mittels eines "Phosphor-Imager" (Molecular Dynamics, Heidelberg, Typ 445 SI) durchgeführt. Die Ergebnisse sind in Figur 11 dargestellt. Expression von Endonexin-short inhibiert konzentrationsabhängig und deutlich die Aktivierung des uPAR-Promotors. Dies zeigt, dass die Expression von uPAR-Rezeptoren konzentrationsabhängig von der Gegenwart von β3-Endonexin-short abhängt, was dessen Bedeutung im Prozess der Zellmigration und damit bei der Plaquedestabilisierung unterstreicht.

### Beispiel 11

### Freisetzung von MCP-1 aus menschlichen Endotheizellen

Kultivierte HUVEC-Zellen wurden entweder mit einem β3-Endonexin-short- oder β3-Endonexin-long-kodierenden Plasmid-Konstrukt wie unter Beispiel 5 und 6 oder einem leeren Kontrollvektor transfiziert. 24 Stunden später wurden die Zellen entweder mit Vehikel oder 100 pg/ml Interleukin-1β über 120 Minuten inkubiert. Dann wurde der Überstand abgesaugt, 10 Min. bei 4.000 UpM zentrifugiert und bei -80°C aufbewahrt. Die Konzentrationen von Monozyten-chemotaktischem Protein 1 (MCP-1) wurden mittels ELISA (R&D Systems, Wiesbaden, Bestellnummer DCP00) gemäß Herstellerangaben mit einer Nachweisgrenze von 5 pg/ml bestimmt. Die Ergebnisse (Figur 12) zeigen, dass die Überexpression von β3-Endonexin-short sowohl die basale als auch die Interleukin-induzierte-Freisetzung des atherogenen MCP-1 senkt und folglich atherosklerotische Prozesse günstig beeinflussen kann.

### Beispiel 12

### β3-Endonexin-long erhöht die Dichte von Vitronektinrezeptoren und vermindert deren Internalisierung auch in humanen Endothelzellen

Die Experimente der Figur 13 zeigen analog zu den Ergebnissen in transfizierten CHO-Zellen auch in humanen Endothelzellen, dass nach Überexpression von β3-Endonexin-long die Dichte der Vitronektinrezeptoren deutlich zunimmt. Dies deutet klar darauf hin, dass En-L eine Verminderung der Internalisierung von β3-Integrinen auch an Endothelzellen bewirken kann.

### Beispiel 13

### β3-Endonexin-short interagiert direkt mit dem Transkriptionsfaktor p65 von NF-kB und verhindert so die Zytokin-induzierte Stimulation von NF-kB

Die Experimente der Figuren 14 und 15 zeigen, dass En-S und En-L direkt mit dem nukleären Transkriptionsfaktor NF-kB interagieren. Diese Bindung vermindert die Aktivierbarkeit dieses Faktors durch Zytokine deutlich, wie anhand der klaren Verminderung der NF-kB-Bindungsaktivität im Gel gut erkennbar ist.

### Beispiel 14

### β3-Endonexin-long, nicht β3-Endonexin-short, ist das dominante Protein in menschlichen Thrombozyten und Endothelzellen

Figur 16 zeigt, dass nicht wie bisher angenommen, β3-Endonexin-short, sondern β3-Endonexin-long das dominante Protein in menschlichen Thrombozyten und Endothelzellen ist. Anhand der Ergebnisse an den transfizierten CHO-Zellen lässt sich folgern, dass die Internalisierung von thrombozytären Fibrinogenrezeptoren durch dieses Protein gehemmt wird.

## Patentansprüche

1. Verwendung von β3-Endonexin-long oder β3-Endonexin-short zum Auffinden von Wirkstoffen zur Behandlung von Arteriosklerose, daraus resultierenden instabilen Plaques, akuter Koronarthrombose, Herzinfarkt, Schlaganfall, peripheren arteriellen Verschlußkrankheiten, chronischem venösen Ulcus und Restenosierungsprozessen.

2. Verfahren zur Identifizierung von Verbindungen, die eine Bindung von β3-Endonexin-short an β3-Integrin hemmen, als Wirkstoffe zur Behandlung von Arteriosklerose, daraus resultierenden instabilen Plaques, akuter Koronarthrombose, Herzinfarkt, Schlaganfall, peripheren arteriellen Verschtußkrankheiten, chronischem venösen Ulcus und Restenosierungsprozessen, **gekennzeichnet durch** die folgenden Schritte:
(a) Inkubation eines Gemisches enthaltend
(a1) β3-Endonexin-short oder ein hierzu in Bezug auf die Bindung an β3-Integrin funktionsäquivalentes Peptid oder Polypeptid;
(a2) β3-Integrin oder ein hierzu in Bezug auf die Bindung an β3-Endonexin-short funktionsäquivalentes Peptid oder Polypeptid;
(a3) eine zu testende Verbindung; und
(b) Nachweis der Hemmung einer Bindung der Komponente (a1) an die Komponente (a2) bei Anwesenheit der Verbindung (a3) im Vergleich zur Abwesenheit der Verbindung (a3).

3. Verfahren zur Identifizierung von Verbindungen, die einen Einfluss von β3-Endonexin-long auf β3-Integrin hemmen als Wirkstoffe zur Behandlung von Arteriosklerose, daraus resultierenden instabilen Plaques, akuter Koronarthrombose, Herzinfarkt, Schlaganfall, peripheren arteriellen Verschlußkrankheiten, chronischem venösen Ulcus und Restenosierungsprozessen, **gekennzeichnet durch** die folgenden Schritte:
(a) Inkubation eines Gemisches enthaltend
(a1) β3-Endonexin-long oder eine hierzu in Bezug auf die Bindung an β3-Integrin funktionsäquivalentes Peptid oder Polypeptid;
(a2) β3-Integrin oder ein hierzu in Bezug auf die Bindung an β3-Endonexin-long funktionsäquivalentes Peptid oder Polypeptid;
(a3) eine zu testende Verbindung;
(b) Nachweis der Hemmung eines Einflusses der Komponente (a1) auf die Komponente (a2) bei Anwesenheit der Verbindung (a3) im Vergleich zur Abwesenheit der Verbindung (a3).

4. Verfahren zur Identifizierung von Verbindungen, die die Konkurrenz der Bindung von β3-Integrin an β3-Endonexin-short und β3-Endonexin-long verschieben, als Wirkstoffe zur Behandlung von Arteriosklerose, daraus resultierenden instabilen Plaques, akuter Koronarthrombose, Herzinfarkt, Schlaganfall, peripheren arteriellen Verschlußkrankheiten, chronischem venösen Ulcus und Restenosierungsprozessen, **gekennzeichnet durch** die folgenden Schritte:
(a) Inkubation eines Gemisches enthaltend
(a1) β3-Endonexin-short oder ein hierzu in Bezug auf die Bindung an β3-Integrin funktionsäquivalentes Peptid oder Polypeptid;
(a2) β3-Endonexin-long oder ein hierzu in Bezug auf die Bindung an β3-Integrin funktiönsäquivalentes Peptid oder Polypeptid;
(a3) β3-Integrin oder ein hierzu in Bezug auf die Bindung an β3-Endonexin-short und/oder β3-Endonexin-long funktionsäquivalentes Peptid oder Polypeptid;
(a4) eine zu testende Verbindung; und
(b) Nachweis einer Verschiebung des Verhältnisses der Bindung der Komponente (a1) an die Komponente (a3) zur Bindung der Komponente (a2) an die Komponente (a3) im Vergleich zur Abwesenheit der zu testenden Verbindung (a4).

## Claims

1. Use of β3-endonexin-long or β3-endonexin-short for finding active substances for the treatment of arteriosclerosis, unstable plaques resulting from the latter, acute coronary thrombosis, cardiac infarct, stroke, peripheral arterial occlusion diseases, chronic venous ulcer and restenosing processes.

2. A method of identifying compounds that inhibit binding of β3-endonexin-short to β3-integrin, as active agents for the treatment of arteriosclerosis, unstable plaques resulting from the latter, acute coronary thrombosis, cardiac infarct, stroke, peripheral arterial occlusion diseases, chronic venous ulcer and restenosing processes, said method being **characterised by** the following steps:
(a) incubation of a mixture comprising
(a1) β3-endonexin-short or a peptide or polypeptide that is functionally equivalent thereto with regard to binding to β3-integrin;
(a2) β3-integrin or a peptide or polypeptide that is functionally equivalent thereto with regard to binding to β3-endonexin-short;
(a3) a compound to be tested; and
(b) detection of the inhibition of the binding of component (a1) to component (a2) in the presence of compound (a3) in comparison with the absence of compound (a3).

3. A method of identifying compounds that inhibit an influence of β3-endonexin-long on β3-integrin, as active agents for the treatment of arteriosclerosis, unstable plaques resulting from the latter, acute coronary thrombosis, cardiac infarct, stroke, peripheral arterial occlusion diseases, chronic venous ulcer and restenosing processes, said method being **characterised by** the following steps:
(a) incubation of a mixture comprising
(a1) β3-endonexin-long or a peptide or polypeptide that is functionally equivalent thereto with regard to binding to β3-integrin;
(a2) β3-integrin or a peptide or polypeptide that is functionally equivalent thereto with regard to binding to β3-endonexin-long;
(a3) a compound to be tested; and
(b) detection of the inhibition of an influence of component (a1) an component (a2) in the presence of compound (a3) in comparison with the absence of compound (a3).

4. A method of identifying compounds that shift the competition of the binding of β3-integrin to β3-endonexin-short and β3-endonexin-long, as active agents for the treatment of arteriosclerosis, unstable plaques resulting from the latter, acute coronary thrombosis, cardiac infarct, stroke, peripheral arterial occlusion diseases, chronic venous ulcer and restenosing processes, said method being **characterised by** the following steps:
(a) incubation of a mixture comprising
(a1) β3-endonexin-short or a peptide or polypeptide that is functionally equivalent thereto with regard to binding to β3-integrin;
(a2) β3-endonexin-long or a peptide or polypeptide that is functionally equivalent thereto with regard to binding to β3-integrin;
(a3) β3-integrin or a peptide or polypeptide that is functionally equivalent thereto with regard to binding to β3-endonexin-short and/or to β3-endonexin-long;
(a4) a compound to be tested; and
(b) detection of a shift in the ratio of the binding of component (a1) to component (a3) to the binding of component (a2) to component (a3) in comparison with the absence of the compound to be tested (a4).

## Revendications

1. Utilisation de la β3-endonexine longue ou de la β3-endonexine courte pour trouver des substances actives pour le traitement de l'artériosclérose, de plaques instables résultant de cette dernière, de la thrombose coronaire aiguë, de l'infarctus du myocarde, de l'ictus, des maladies par occlusion artérielle périphérique, de l'ulcère veineux chronique et des processus dé resténose.

2. Procédé pour identifier des composés qui inhibent la liaison de la β3-endonexine courte à la β3-intégrine, en tant qu'agents actifs pour le traitement de l'artériosclérose, de plaques instables résultant de cette dernière, de la thrombose coronaire aiguë, de l'infarctus du myocarde, de l'ictus, des maladies par occlusion artérielle périphérique, de l'ulcère veineux chronique et des processus de resténose, ledit procédé étant **caractérisé par** les étapes suivantes :
(a) incubation d'un mélange contenant
(a1) de la β3-endonexine courte ou un peptide ou polypeptide qui lui est fonctionnellement équivalent en ce qui concerne la liaison à la β3-intégrine ;
(a2) de la β3-intégrine ou un peptide ou polypeptide qui lui est fonctionnellement équivalent en ce qui concerne la liaison à la β3-endonexine courte ;
(a3) un composé devant être testé ; et
(b) détection de l'inhibition d'une liaison du composant (a1) au composant (a2) en présence du composé (a3) par comparaison avec le cas où il y a absence du composé (a3).

3. Procédé pour identifier des composés qui inhibent l'influence de la β3-endonexine longue sur la β3-intégrine, en tant qu'agents actifs pour le traitement de l'artériosclérose, de plaques instables résultant de cette dernière, de la thrombose coronaire aiguë, de l'infarctus du myocarde, de l'ictus, des maladies par occlusion artérielle périphérique, de l'ulcère veineux chronique et des processus de resténose, ledit procédé étant **caractérisé par** les étapes suivantes :
(a) incubation d'un mélange contenant
(a1) de la β3-endonexine longue ou un peptide ou polypeptide qui lui est fonctionnellement équivalent en ce qui concerne la liaison à la β3-intégrine ;
(a2) de la β3-intégrine ou un peptide ou polypeptide qui lui est fonctionnellement équivalent en ce qui concerne la liaison à la β3-endonexine longue ;
(a3) un composé devant être testé ; et
(b) détection de l'inhibition de l'influence du composant (a1) sur le composant (a2) en présence du composé (a3) par comparaison avec le cas où il y a absence du composé (a3).

4. Procédé pour identifier des composés qui décalent la compétition de liaison de la β3-intégrine à la β3-endonexine courte et à la β3-endonexine longue, en tant qu'agents actifs pour le traitement de l'artériosclérose, de plaques instables résultant de cette dernière, de la thrombose coronaire aiguë, de l'infarctus du myocarde, de l'ictus, des maladies par occlusion artérielle périphérique, de l'ulcère veineux chronique et des processus de resténose, ledit procédé étant **caractérisé par** les étapes suivantes :
(a) incubation d'un mélange contenant
(a1) de la β3-endonexine courte ou un peptide ou polypeptide qui lui est fonctionnellement équivalent en ce qui concerne la liaison à la β3-intégrine ;
(a2) de la β3-endonexine longue ou un peptide ou polypeptide qui lui est fonctionnellement équivalent en ce qui concerne la liaison à la β3-intégrine ;
(a3) de la β3-intégrine ou un peptide ou polypeptide qui lui est fonctionnellement équivalent en ce qui concerne la liaison à la β3-endonexine courte et/ou la β3-endonexine longue ;
(a4) un composé devant être testé ; et
(b) détection d'un décalage dans le rapport de la liaison du composant (a1) au composant (a3) à la liaison du composant (a2) au composant (a3) par comparaison avec le cas où il y a absence du composé devant être testé (a4).
